# FASCICULE DE BREVET EUROPEEN

(11) **EP 2 276 418 B1**
(45) Date de publication et mention de la délivrance du brevet: **01.06.2016**
(21) Numéro de dépôt: 08737386.6
(22) Date de dépôt: 07.04.2008
(51) Int. Cl.: A61C 19/04

(54) **DISPOSITIF POUR DETERMINER LA POSITION APICALE DANS UN CANAL DE RACINE DENTAIRE**
VORRICHTUNG ZUR BESTIMMUNG DER APIKALEN POSITION IN EINEM ZAHNWURZELKANAL
DEVICE FOR DETERMINING THE APICAL POSITION IN A DENTAL ROOT CANAL

(43) Date de publication de la demande: 26.01.2011
(73) Titulaire: Maillefer Instruments Holding S.À.R.L., 1338 Ballaigues (CH)
(72) Inventeur: AEBY, François, CH-1442 Montagny (CH); FARINE, Pierre-André, CH-2000 Neuchâtel (CH); MERZ, Roman, CH-2000 Neuchâtel (CH); BOTTERON, Cyril, CH-3238 Gals (CH)
(74) Mandataire: Mayjonade, Bernard
(86) Numéro de dépôt international: PCT/IB2008/000820
(87) Numéro de publication internationale: WO 2009/125237

(56) Documents cités:
- WO-A-01/47414
- US-A- 5 080 586
- US-A- 5 096 419
- US-A- 6 059 569

## Description

La présente invention concerne le domaine des procédés et dispositifs de localisation d'apex, utilisés en endodontie, pour repérer, dans un canal de racine d'une dent, la position en profondeur de l'apex, c'est-à-dire la position du sommet de cette racine *(i.e. terminus apical*) et plus précisément l'extrémité de l'orifice du *foramen apical* au fond du canal radiculaire.

Les localisateurs d'apex servent lors des opérations de chirurgie dentaire, notamment lors d'une opération de nettoyage et de mise en forme du canal radiculaire, pour éviter de franchir le *foramen apical*, *i.e.* dépasser le *terminus apical* et se garder d'atteindre le ligament maxillaire sous-jacent avec ses faisceaux nerveux.

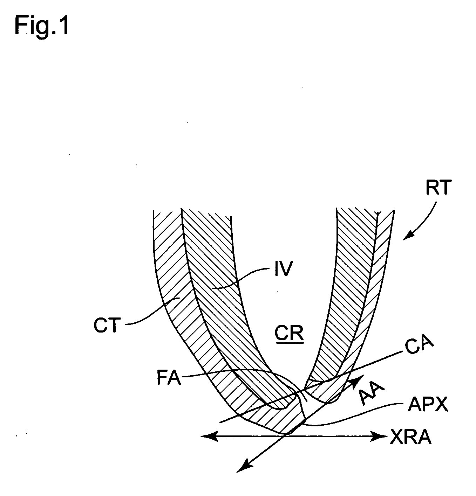

La figure 1 montre la structure anatomique d'une dent schématisée selon un plan de coupe suivant l'axe du canal d'une racine de la dent. Certaines dents, telles que les molaires et prémolaires, peuvent comporter plusieurs racines RT ou du moins plusieurs canaux radiculaires CR qui peuvent être distincts ou accolés.

L'extrémité de la racine RT est percée d'un orifice FA dit *foramen apical* pour le passage des faisceaux nerveux et des vaisseaux. Parfois, comme illustré sur la figure 1, cet orifice FA à l'extrémité du canal radiculaire CR se rétrécit en formant un goulot d'étranglement au niveau de la *constriction apicale* CA (goulot étroit permettant le passage du paquet vasculo-nerveux irriguant la pulpe). Dans d'autres cas (non représenté), le canal radiculaire présente une section large, sans rétrécissement.

Au niveau de cette constriction apicale CA, se situe l'interface de jonction cementino-dentinaire CT/IV, interface entre des substances minérales (cément/ivoire) qui présentent des propriétés électriques contrastées.

Lors d'opérations de chirurgie endodontique, comme les opérations de nettoyage et de mise en forme du canal dentaire CR, le dentiste cherche à éliminer tous les matériaux, débris et fluides organiques qui emplissent le canal radiculaire CR jusqu'au fond, c'est-à-dire jusqu'à l'extrémité du *foramen apical* FA, pour éviter une réapparition d'un abcès dentaire dans le canal radiculaire CR.

Toutefois, son objectif est surtout, autant que possible, de ne pas dépasser le *terminus apical* APX, d'une part, pour ne pas occasionner de douleur au patient, et, d'autre part, pour ne pas creuser une cavité sous la racine, au-delà de l'apex, ce qui pourrait donner lieu à l'apparition d'un abcès.

II est donc de la plus haute importance pour le dentiste de localiser très précisément le *foramen* FA et le *terminus apical* APX.

Comme indiqué sur la figure 1, les clichés radiographiques des dents, pris selon le plan horizontal XRA du maxillaire donnent généralement une position radiographique de l'apex incorrecte qui ne correspond pas à la véritable position du plan directeur de l'apex anatomique AA.

Des dispositifs électroniques de localisation d'apex ont été développés depuis près d'un demi-siècle pour localiser précisément l'extrémité du canal radiculaire en s'appuyant sur les changements de propriétés électriques dans cette zone de transition.

Les premières générations de localisateurs d'apex, développées par Sunada sur la base des travaux du Pr. Suzuki, mettent en oeuvre un principe de mesure de résistance du canal radiculaire, en se basant sur le constat que, lors du franchissement de la zone apicale, la valeur de résistance chute brusquement et franchit un seuil de résistance d'environ R = 6,5 kΩ valeur sensiblement constante d'un individu à l'autre.

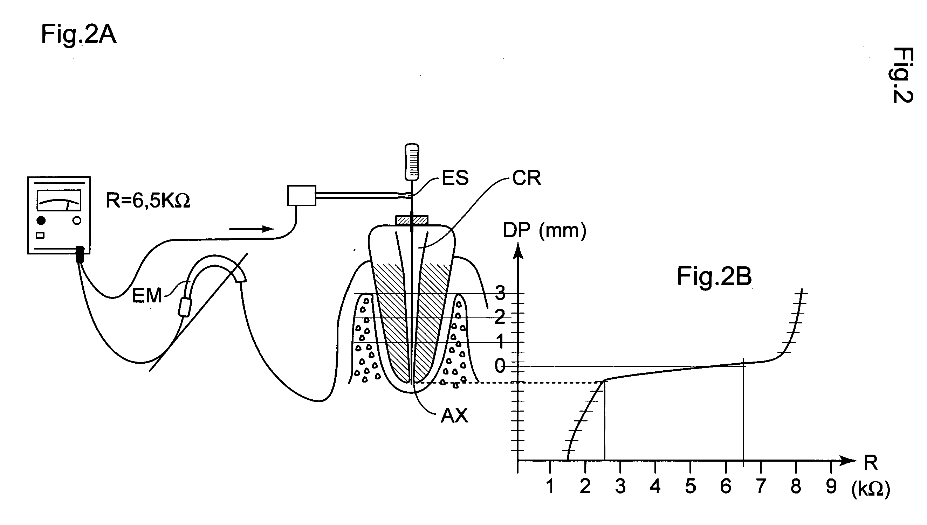

Comme illustré sur la figure 2A, la résistance est mesurée entre une première électrode ES formée par une lime ou sonde endodontique insérée dans le canal radiculaire CR et une seconde électrode EM conformée pour être mise en contact étroitement conducteur électriquement avec une muqueuse de la bouche (lèvre, gencive...).

La figure 2B indique que la résistance R diminue d'abord faiblement à mesure que la lime endodontique ES est enfoncée en profondeur DP au sein du canal radiculaire CR dans l'axe de la dent, puis R chute brutalement lors de la traversée de la zone apicale, avant de se rétablir à une valeur plancher une fois le *terminus apical* dépassé.

Sunada a établi que l'apex se situe dans la zone où la résistance franchit la valeur de seuil R = 6,5 kΩ, valeur sensiblement constante d'un individu à l'autre.

Le brevet US-5,096,419 au nom de Kobayashi de la société MORITA, fait état de deux documents japonais de l'art antérieur JP 2817/62 et JP 25381/62 portant sur deux lignées d'appareils de mesure permettant de localiser la position de l'apex et de déterminer la profondeur du canal radiculaire.

La première lignée d'appareils est basée sur un principe de mesure de résistance, en courant continu, la résistance en continu chutant brusquement lors du franchissement de la zone apicale.

La seconde lignée d'appareils est basée sur un principe de mesure d'impédance, généralisation de la résistance, mais mesurée en signal alternatif et intégrant deux composantes résistive et capacitive ; l'impédance en signal alternatif chute quand la pointe de la sonde approche de l'apex.

Le premier principe de mesure de résistance permet seulement de détecter le dépassement du *terminus apical,* ce qui ne satisfait pas l'objectif du dentiste d'être prévenu avant d'avoir dépassé l'apex.

Le second principe de mesure d'impédance devrait s'avérer plus prévenant, car l'impédance est sensée chuter lors du changement de propriétés à l'interface cément/dentine CT/IV lors du franchissement de la *constriction apicale* CA qui se situe avant d'atteindre la ligne directrice AA de l'apex comme l'indique la figure 1.

Premier inconvénient, ce second principe de mesure basé sur la détection de chute d'impédance à la jonction cément/dentine de la *constriction apicale* ne fonctionne pas sur des enfants et des jeunes patients car leurs dents n'ont pas ou peu de dentine hyperminéralisée.

De façon générale, ces deux lignées d'appareils nécessitent des opérations délicates d'étalonnages et de calibration, opérations imprécises, fastidieuses et source d'erreurs.

En pratique, plus généralement, ces deux lignées de localisateurs d'apex, ont l'inconvénient d'indiquer la position de l'apex seulement après que la pointe de l'électrode-sonde a franchi la *constriction apicale.* Les mesures de résistance ne chutent pas avant que la pointe de la sonde ait dépassé le *terminus apical* APX. II s'avère en fait que les mesures d'impédance chutent seulement lorsque la pointe de la lime a dépassé l'orifice du *foramen apical* FA et touche le ligament sous la racine dentaire RT. Or, les dentistes cherchent à ne surtout pas franchir le *foramen apical* FA.

Autre problème important, les deux principes de mesure de ces deux lignées de localisateurs d'apex ont pour inconvénient que les mesures de résistance/conductance deviennent complètement imprécises, voire aberrantes, en présence de fluides conducteurs dans le canal radiculaire.

Or, lors des opérations de nettoyage et de mise en forme dentaire, le canal est généralement rempli de fluides et de matériaux, notamment de matières et corps organiques (salive, sang, lymphe, sérum, fluides physiologiques, débris organiques) qui se comportent comme des milieux légèrement salés donc moyennement conducteurs, analogues à ce qu'on appelle le sérum ou liquide physiologique (solution aqueuse saline commune de NaCl à 0,9%) qui est une solution ionique modérément conductrice, comme l'eau de mer.

En outre, les dentistes sont tenus de nettoyer en continu la bouche du patient avec un flux de liquide de rinçage à base de solution saline de NaCl, conductrice et surtout avec des solutions désinfectantes, notamment de liquide de Dakin ("soluté neutre dilué d'hypochlorite de sodium", NaClO dilué à 2,5% ou 5%, apparenté à la véritable eau de Javel) qui est une solution ionique très fortement conductrice (ions OH-). De telles solutions ioniques fortement conductrices perturbent totalement les mesures de conductivité (résistance, impédance) et faussent entièrement toute détermination de la position de l'apex.

La présence courante de telles solutions et fluides organiques, lors des opérations dentaires, prétéritent l'utilisation des appareils de localisation d'apex basés sur de tels principes de mesure de résistance ou de conductance.

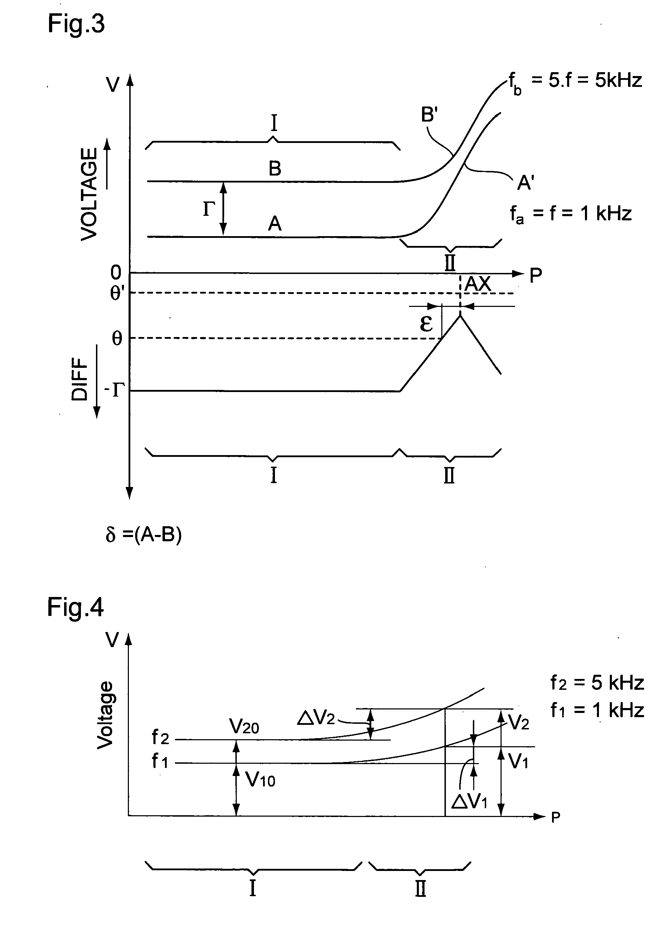

Le perfectionnement à cet état de la technique proposé par Kobayashi dans le document US-5,096,419 consiste, pour s'affranchir des fluctuations de conductivité apportées par la présence de solutions ioniques, à confronter deux mesures de conductance effectuées à deux fréquences distinctes f et 5f comme illustré sur la figure 3.

Selon ce troisième principe de mesure, on effectue des mesures de tension (V) aux bornes d'une résistance de référence R = 5kΩ placée en série avec les électrodes. Le circuit série est alimenté par un générateur de signal carré à la fréquence f, ce qui produit des composantes de signaux harmoniques aux fréquences fa = f et fb = 5f. Dans un premier temps (phase I), au cours de l'insertion de la sonde dans le canal radiculaire, les mesures de tension A et B relevées aux deux fréquences fa=f et fb= 5f restent stables. Dans un second temps (phase II), les deux mesures de tension A' et B' augmentent à la traversée d'une zone II correspondant à la constriction apicale (car l'impédance du canal chute à l'approche de la jonction cément/dentine).

D'après le document US-5,096,419 de Kobayashi les deux courbes A' et B' ne sont pas équidistantes dans la zone II mais leur écart r diminue.

Selon Kobayashi, la différence δ entre les deux mesures de tension A et B (δ=A-B) d'abord de valeur -Γ sensiblement constante dans la zone I diminuerait dans la zone II.

Kobayashi précise que dans la zone II, l'écart B-A ou la différence δ=A-B se rapproche d'une valeur d'extremum (écart minimum) avant de s'infléchir brutalement dans l'autre sens et de s'écarter à nouveau. L'extremum, c'est-à-dire le point AX ou l'écart IB-AI est minimum (i.e. δ=A-B max.) correspond à la position de l'apex, d'après l'enseignement de US-5,096,419.

Le document US-5,096,419 décrit, par suite, un circuit électronique de détection de seuil sophistiqué, pour déterminer à quel point la différence δ=A-B entre les deux tensions mesurées aux deux fréquences fa = f = 1kHz et fb = 5f = 5kHz franchit une valeur de seuil θ correspondant à la position de l'apex.

L'inconvénient de ce dispositif est que la fixation de la valeur de seuil θ nécessite des opérations de calibration toujours délicates, imprécises et sources d'erreurs. En pratique, l'allure précise des variations des courbes A et B et leur écart |δ|=|A-B| sont éminemment variables selon les individus et les conditions électriques régnant dans chaque canal radiculaire.

Pour chaque canal individuel de chaque racine de chaque dent, il faut recommencer les opérations d'étalonnage et de calibration, opérations spécialisées, longues et fastidieuses pour le dentiste, ce qui le détourne de ces appareillages.

En effet, selon que le seuil est fixé à une valeur θ en deçà de l'extremum ou à une valeur θ' au delà de l'extremum, soit la mesure de la position P de l'apex AX est imprécise et entachée d'une erreur ε, soit carrément on ne détecte pas de franchissement de seuil et le dispositif ne signale pas que la sonde dépasse le *terminus apical.*

De façon générale, ce troisième principe de détection de différence de mesures établies à deux fréquences a encore l'inconvénient de ne pas fixer un critère de mesure absolu de la position de l'apex.

La détection du franchissement d'un seuil à toujours l'inconvénient d'être relative à la fixation d'une valeur de seuil arbitraire.

D'un autre point de vue, si on cherchait à détecter le point AX de rebroussement, c'est-à-dire le point d'inflexion AX où la courbe δ=A-B atteint l'extremum et change de sens de variation, ce qui constituerait un critère absolu, il serait quand même nécessaire de dépasser le point AX, c'est-à-dire de franchir le *terminus apical* pour détecter le passage à *l'extremum* et le changement du sens de variation.

La figure 4 illustre un quatrième principe de localisation d'apex proposé par le document US-5'080'586 au nom de Kawai de l'Institut OSADA.

Le document US-5'080'586 décrit un système de mesure comparable à celui du document US-5'096'419 et consistant à appliquer deux tensions alternatives V₁ et V₂ ayant deux fréquences distinctes f₁ et f₂ aux bornes d'un circuit comprenant deux électrodes (une aiguille insérée dans le canal radiculaire d'une dent et une électrode au contact d'une muqueuse buccale) en série avec une résistance de mesure.

Il se trouve que les deux fréquences f₁ = 1 kHz et f₂ = 5 kHz proposées par le document US - 5,080,586 sont identiques aux deux fréquences fa = f = 1 kHz et fb = 5f = 5kHz utilisés selon l'enseignement de l'autre document US-5'096'419.

Par contre, selon la figure 4 qui reprend l'allure des courbes de mesure du document US-5'080'586 de l'art antérieur, les courbes de mesure des deux tensions V₁ et V₂ relevées aux deux fréquences f₁ = 1 kHz et f₂ = 5 kHz, divergent et s'écartent continûment avec la profondeur P d'insertion de l'électrode, l'écart (V₂-V₁) croissant de façon monotone.

Les points de vue sur l'allure des courbes de tension relevées aux deux fréquences de f = 1 kHz et 5f = 5 kHz sont donc divergents et illustrent à quel point les mesures sont capricieuses, peu fiables et ne constituent pas un critère de mesure absolu pour déterminer avec précision la position de l'apex.

Pour déterminer la position de l'apex, le document US-5'080'586 propose d'établir le rapport entre ces deux tensions V1 et V2 relevées aux deux fréquences f₁ et f₂ (ratio V2/V1) et d'établir une valeur seuil, la position de l'apex A correspondant au franchissement de ce seuil par le ratio V2/V1.

Ce principe alternatif de mesure a toujours l'inconvénient de ne pas constituer un critère absolu pour déterminer la position exacte de l'apex mais de se référer à des valeurs de seuils relatives, variant selon les individus et les conditions électrolytiques régnant dans chaque canal radiculaire, ce qui demande au dentiste des opérations de calibration délicates, imprécises et source d'erreurs.

Plus généralement, ces dernières générations de localisateurs d'apex sont basées sur des principes de mesure de tension aux bornes d'une résistance de référence en série avec les deux électrodes qui reflètent la conductance (inverse de l'impédance) existant dans le canal radiculaire entre les électrodes.

Le problème est que de tels principes de mesure sont directement affectés par la présence de fluides conducteurs dans le canal radiculaire qui faussent entièrement la détermination de la position de l'apex.

Or, comme mentionné précédemment, la présence de fluides conducteurs dans le canal radiculaire est inévitable lors des opérations de chirurgie dentaire, du fait de la présence de fluides et de matières organiques (sang, lymphe, salive, sérum, débris organiques), et de la nécessité de nettoyer la bouche avec des solutions de rinçage (liquide physiologique, *i*.*e*. solution de NaCl à 0,9%) ou avec des solutions désinfectantes (liquide de Dakin, *i.e.* solution de NaClO).

Par ailleurs, un autre problème général des dispositifs de localisation d'apex basés sur des mesures d'impédance du canal radiculaire de la dent est qu'ils ne permettent pas de résoudre les dents complexes comportant plusieurs canaux radiculaires ou des canaux radiculaires présentant des bifurcations ou des aberrations (racines multiples, fourchues, ramifiées ou jumelles, excroissances...).

Les molaires comptent plusieurs racines et canaux radiculaires généralement bien séparés. Les prémolaires et molaires comportent généralement des racines jumelles, juste subdivisées à leur extrémité par une bifurcation en deux canaux radiculaires jumeaux (fourchus). D'autres dents peuvent comporter des ramifications ou des aberrations. Or, les dents généralement les plus concernées par les opérations de chirurgie dentaire et de nettoyage et de mise en forme endodontique sont précisément ces dents complexes, notamment les molaires et prémolaires.

L'objet de l'invention est donc de prévoir un moyen de localisation d'apex résolvant ces problèmes et remédiant aux inconvénients des localisateurs d'apex de l'état de la technique.

L'objectif de l'invention est de développer des moyens de détection de la position de l'apex se basant sur un critère de mesure absolu de la profondeur de l'apex, s'affranchissant de la fixation arbitraire de seuils relatifs et dépendant du patient ou des fluctuations des caractéristiques du canal radiculaire.

L'objectif de la présente est donc de réaliser un système ou dispositif de localisation d'apex permettant de déterminer la position de l'apex avec une bonne précision quelle que soit la configuration ou les conditions présentées par les canaux radiculaires des dents.

L'objectif est en particulier de parvenir à déterminer précisément la position de l'apex en étant aussi peu sensible que possible à la présence de fluides conducteurs, et notamment de solutions de rinçage, de corps organiques ou de liquides physiologiques analogues à la présence de solution saline commune à base de chlorure de sodium (NaCl à 0,9%) ainsi que de solutions ioniques désinfectantes telles que le liquide de Dakin à base d'hypochlorite de sodium (NaClO à 2,5% ou 5%) utilisé systématiquement lors des opérations de chirurgie dentaire, notamment pour les opérations de curage d'abcès endodontique.

Un autre objectif est de réaliser un dispositif de localisation d'apex permettant la résolution de dents à canaux radiculaires complexes, c'est-à-dire permettant de distinguer, discriminer et déterminer la position de l'extrémité de chaque canal radiculaire de la dent avec une bonne précision, autant que possible meilleure que les dispositifs existants antérieurement dans le commerce.

Un objectif particulier est de réaliser un système de localisation d'apex donnant une détermination de la position de l'apex avec non seulement peu d'erreur par rapport à la position réelle du *terminus apical*, mais permettant en outre que la position de l'apex soit signalée avant d'atteindre ou de dépasser le *terminus apical.*

Succinctement, l'invention, telle que définie par les revendications, prévoit un dispositif endodontique comportant classiquement deux électrodes, l'une en liaison avec une lime ou une sonde métallique apte à être insérée dans le canal radiculaire d'une dent, l'autre apte à être mise en contact électrique étroitement conducteur à basse impédance avec une muqueuse buccale, les deux électrodes étant incorporées en série dans un circuit alimenté par un générateur de signaux alternatifs à agilité de fréquence et comportant un montage de mesure d'amplitude des signaux alternatifs. Le montage comporte notamment un appareil de mesure d'amplitude en tension des signaux alternatifs aux bornes d'une résistance de référence de mesure, en série avec les électrodes et le générateur à agilité de fréquences alimentant ce circuit-série, ce qui revient à mesurer l'amplitude du courant alternatif traversant la résistance de référence et les électrodes. Selon l'invention, l'amplitude des signaux alternatifs est relevée à des fréquences très éloignées appartenant à des bandes de fréquences opposées comme le permet l'étendue des gammes fréquentielles du générateur à agilité de fréquence.

D'après l'invention, les niveaux d'amplitudes des signaux relevés à deux fréquences déterminées se croisent au passage de la constriction apicale, c'est-à-dire qu'en fonction et au fur et à mesure de l'insertion en profondeur de l'électrode-sonde dans un canal radiculaire d'une dent, on distingue plusieurs phases, zones et/ou hiérarchies, comme suit:
- initialement, lors de l'introduction de la sonde, au début de la partie coronaire de la dent (« couronne »), le premier niveau d'amplitude de signal relevé à une fréquence inférieure (basse fréquence f) est nettement supérieur à l'autre niveau d'amplitude relevé à la fréquence supérieure (haute fréquence F), puis,
- au cours de l'enfoncement de la sonde dans le canal radiculaire de la dent, les deux niveaux d'amplitude des signaux relevés aux deux fréquences déterminées (fréquences opposées, basse et haute f & F) augmentent en s'approchant de l'extrémité du canal radiculaire,
- dans une zone de transition, qui se trouve correspondre au passage de la constriction apicale, à l'extrémité du canal radiculaire, le second niveau d'amplitude relevé à la fréquence supérieure (haute fréquence F) se rapproche, rejoint et devient sensiblement égal jusqu'à coïncider exactement avec le premier niveau d'amplitude relevé à la fréquence inférieure (basse fréquence f) et éventuellement (si on va au-delà),
- après le dépassement de la zone de constriction apicale, le second niveau d'amplitude de signal relevé à la fréquence supérieure (haute fréquence F) devient supérieur, voire dépasse nettement, le premier niveau d'amplitude relevé à la fréquence inférieure (basse fréquence f).

Bien sûr, pour le dentiste, il est hors de question de chercher à dépasser le *terminus apical* et donc d'atteindre la zone où le second niveau d'amplitude relevé à la fréquence supérieure (haute fréquence F) deviendrait supérieur au premier niveau d'amplitude mesuré à la fréquence inférieure (basse fréquence f).

Ainsi, de façon avantageuse, l'invention fournit un critère de mesure absolue de la position de la constriction apicale, qui correspond au point où les deux niveaux d'amplitude établis aux deux fréquences déterminées (basse et haute fréquences f et F), se croisent et coïncident.

Pour atteindre les objectifs susvisés, il est prévu un procédé de localisation d'apex pour déterminer une mesure de la position en profondeur de l'apex dans un canal de racine d'une dent, mettant en oeuvre un dispositif comprenant une première électrode conductrice formant sonde endodontique apte à être insérée dans le canal radiculaire d'une dent, une seconde électrode conformée pour être mise en contact conducteur électriquement avec une muqueuse buccale, des moyens générateurs de fréquences aptes à produire des signaux électriques alternatifs à plusieurs fréquences, et des moyens de mesure de grandeur de signaux électriques alternatifs dans un circuit comprenant ledit générateur de fréquences, la première électrode-sonde insérée dans le canal radiculaire et la seconde électrode au contact de la muqueuse buccale, le procédé comportant des étapes consistant à :
- exciter le circuit et mesurer les niveaux de grandeur des signaux électriques alternatifs dans le circuit, respectivement, à une fréquence inférieure et à une fréquence supérieure ;
- détecter un point de coïncidence où deux niveaux respectifs de la grandeur électrique mesurée aux dites fréquences inférieure et supérieure se croisent, lesdites fréquences inférieure et supérieure étant suffisamment éloignées pour qu'un tel point de coïncidence existe, ledit point de coïncidence correspondant à la position de l'apex.

De préférence, il est prévu de mesurer des niveaux d'amplitude des signaux électriques appliqués au circuit et plus précisément, de l'intensité du courant traversant les électrodes, notamment en mesurant des valeurs absolues d'amplitude de tension des signaux électriques aux bornes d'une résistance en série avec les électrodes.

Il apparaît, en y prêtant attention, que l'invention met en lumière un fait qui n'était pas manifeste. Curieusement, l'invention se distingue en ce que la fréquence inférieure et la fréquence supérieure sont sélectionnées de sorte que, dans une phase initiale correspondant au commencement de l'insertion de la pointe de l'électrode-sonde endodontique au début du canal radiculaire, le premier niveau mesuré à la fréquence inférieure est supérieur au second niveau mesuré à la fréquence supérieure.

Globalement, d'après l'invention, les fréquences inférieure et supérieure sont sélectionnées dans des bandes de fréquences opposées, distinctes et/ou distantes, i.e. non adjacentes ou encore que lesdites fréquences inférieure et supérieure sont séparées par un ou plusieurs ordres de magnitude. De préférence ladite fréquence supérieure est d'au moins deux, trois ou quatre ordres de magnitude supérieure à ladite fréquence inférieure.

Typiquement, ladite fréquence inférieure est choisie dans une bande de Basses Fréquences tandis que ladite fréquence supérieure est choisie dans une bande de Hautes Fréquences.

En particulier, ladite fréquence inférieure et ladite fréquence supérieure sont situées respectivement dans deux gammes de fréquences opposées de part et d'autre d'une gamme de fréquences incluant au moins la bande conventionnelle numéro quatre (bande n°4 dite T.B.F. ou VLF ou B.O.hm) qui couvre les fréquences de trois kiloHertz à trente kiloHertz (3-30 kHz).

En particulier, il apparaît que la fréquence inférieure est inférieure à 950 Hertz et, de préférence, inférieure à 500 Hertz ; tandis que la fréquence supérieure est supérieure à 9500 Hertz et, de préférence, supérieure à 95 kHz.

Plus précisément, dans des exemples de mode de réalisation de l'invention exposés ci-après, la fréquence inférieure est située dans une bande de fréquence conventionnelle numéro deux ou inférieur, *i.e.* comprise entre 300 Hertz et 30 Hertz ou moins ; tandis que la fréquence supérieure est située dans une bande de fréquence conventionnelle numéro six ou de numéro plus élevé, *i.e.* comprise entre 300 kHz et 3 MHz, voire plus.

Selon un mode particulier de réalisation de l'invention exposé ci-après, la fréquence inférieure est située dans une bande de fréquence d'une dizaine de Hertz à quelques centaines de Hertz, de préférence autour d'une valeur de 100 Hertz, tandis que la fréquence supérieure est choisie dans une bande de fréquence de l'ordre d'un demi ou d'un MégaHertz à cinq ou dix MégaHertz, le choix de la fréquence supérieure pouvant de préférence être réglé sur une valeur choisie parmi un groupe de plusieurs valeurs calibrées autour de {0,5 MHz - 1 MHz - 2 MHz - 5 MHz}, en fonction des conditions électrolytiques régnant dans le canal radiculaire, notamment de la présence de solutions aqueuses ioniques conductrices, telles que la présence de liquide physiologique ou de solution saline commune de chlorure de sodium (NaCl) ou la présence de liquide de Dakin ou de solution désinfectante à base d'hypochlorite de sodium (NaClO).

De façon avantageuse et surprenante, il apparaît, comme exposé dans les résultats exemplaires des campagnes de mesures détaillés ci-après, qu'un tel mode de détection de point de coïncidence, selon l'invention, permet d'obtenir des résultats cohérents de mesure de profondeur d'apex en présence d'électrolytes, notamment en présence de solutions aqueuses ioniques courantes à base de chlorure de sodium (NaCl à ∼1%, précisément 9‰) ainsi qu'en présence de solutions désinfectantes de rinçage utilisées usuellement lors des opérations de chirurgie dentaire, comme les solutions antiseptiques à base d'hypochlorite de sodium (NaClO à 5% ou NaClO à 2,5%).

Autre avantage capital, l'invention permet de résoudre les canaux radiculaires de dents complexes, c'est-à-dire qu'elle permet de distinguer, de discriminer, de déterminer ou donner des résultats de mesure cohérents de la profondeur du canal de chaque racine pour des dents complexes, comme des dents comportant des canaux radiculaires relativement larges ou présentant des bifurcations (racines fourchues, ramifiées ou bifides) ou des aberrations, selon les premiers résultats d'études menées sur des modèles de dents reconstituées et des spécimens de dents réelles.

Le choix des valeurs déterminées des fréquences inf. et sup. permet d'affiner la mesure du point de coïncidence des niveaux d'amplitudes relevés aux dites fréquences (basse fréquence f et haute fréquence F) et d'obtenir qu'il corresponde précisément avec le point de localisation exacte de l'Apex.

En particulier, le choix des fréquences inférieure et supérieure peut être modulé pour optimiser les résultats de mesure, minimiser l'incertitude de mesure et obtenir la meilleure précision en fonction des conditions régnant dans le canal dentaire, notamment selon que le canal radiculaire est irrigué par une solution conductrice à base de chlorure de sodium ("liquide physiologique") ou par une solution ionique à base d'hypochlorite de sodium ("liquide de Dakin") ou selon la configuration du canal radiculaire.

D'autres avantages, caractéristiques et objectifs de l'invention apparaîtront à la lecture de la description d'exemples de mise en oeuvre détaillés ci-après, en regard des planches de dessin annexées, et donnés à titre d'exemples non limitatifs.

Sur les planches de dessin ci-après :
- la figure 1, précédemment évoquée, représente une vue en coupe de la structure du canal d'une racine dentaire avec la position de l'apex à son extrémité, telle qu'elle est connue en anatomie ;
- les figures 2A et 2B, évoquées précédemment, schématisent une mesure en courant continu de la résistance R entre deux électrodes, l'une reliée à une sonde endodontique, l'autre au contact d'une muqueuse buccale et permettant de localiser la profondeur DP de l'apex au point où R=6,5 kOhms, selon un premier principe de localisateur d'apex connu ;
- la figure 3, exposée antérieurement, est un diagramme représentant, en fonction de la profondeur d'insertion d'une sonde, les courbes de niveaux en tension A et B relevées à deux fréquences fa = 1 kHz et fb = 5 kHz ainsi que l'allure de la différence δ entre ces niveaux, qui présente un point d'inflexion (extremum) à l'endroit de l'Apex, selon un autre principe de localisation d'apex utilisé dans un type de dispositif de l'art antérieur ;
- la figure 4, exposée antérieurement, est un autre diagramme représentant d'autres courbes de niveaux en tension V₁ et V₂ relevées aussi aux deux fréquences f1=1 kHz, f2=5 kHz selon un autre principe de localisation d'apex utilisant le rapport des tensions (ratio V2/V1) mis en oeuvre dans un autre type de dispositif selon un autre document de l'art antérieur ;

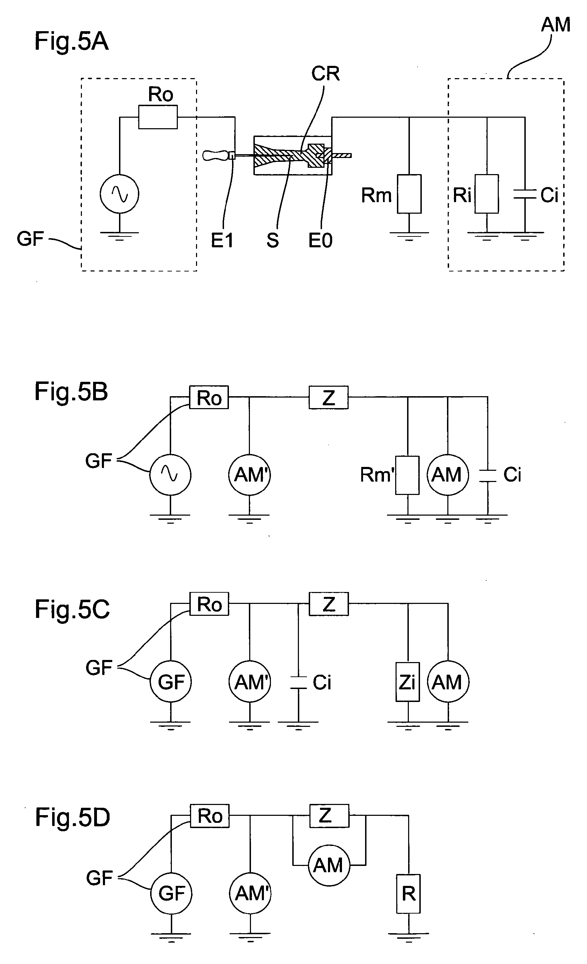

- la figure 5A représente un schéma du circuit électrique du dispositif de localisation d'apex mis en oeuvre selon l'invention ;
- les figures 5B à 5D représentent des schémas équivalents de circuit électrique du dispositif de localisation d'apex selon l'invention ;

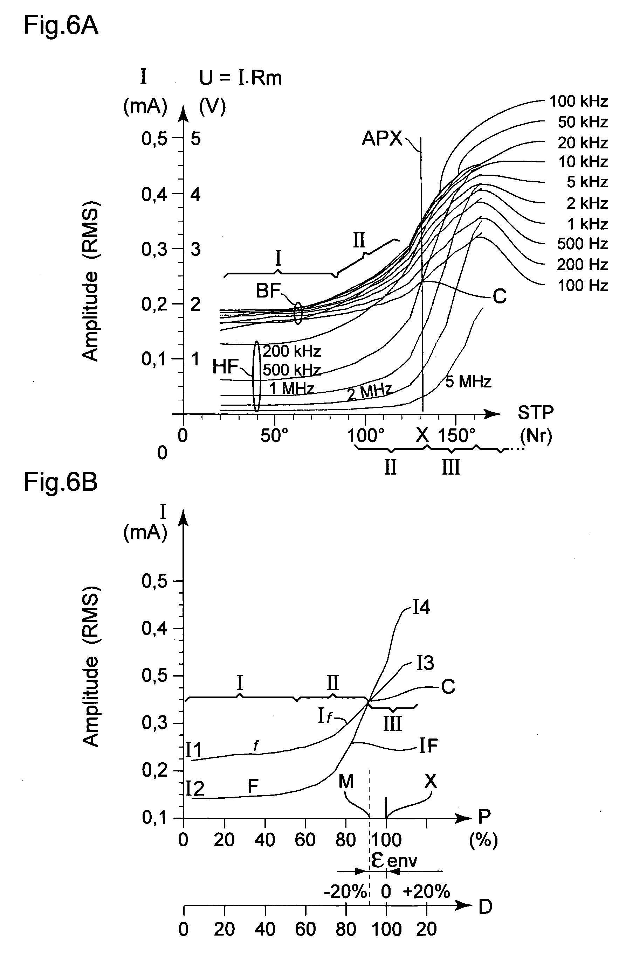

- la figure 6A est un diagramme de mesure d'amplitude en fonction de la profondeur d'insertion de l'électrode-sonde montrant l'allure générale des courbes relevées à différentes fréquences allant de basse fréquence à haute fréquence et leur croisement, selon l'invention ;
- la figure 6B montre un diagramme de courbes extrait de la figure 6A, faisant ressortir le croisement de deux courbes de mesures particulières sélectionnées relevées à deux fréquences opposées, l'une à basse fréquence f=100 Hz, l'autre à haute fréquence F=500 kHz, leur point de croisement C correspondant à la position X de l'apex, selon l'invention ;

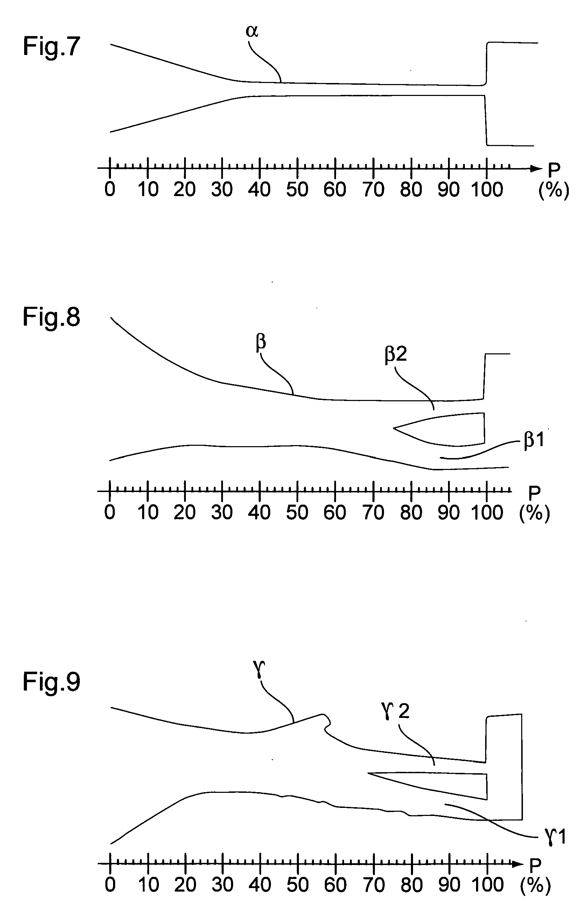

- les figures 7, 8 et 9 montrent trois modèles de racines dentaires α, β et γ (le premier α se rétrécissant en entonnoir avec un canal étroit, le deuxième β présentant une bifurcation en deux canaux radiculaires, le troisième γ présentant des aberrations de canaux ramifiés), sur lesquels des mesures de profondeur de chaque canal radiculaire ont été expérimentées (cf. figures suivantes) avec un dispositif selon l'invention ;

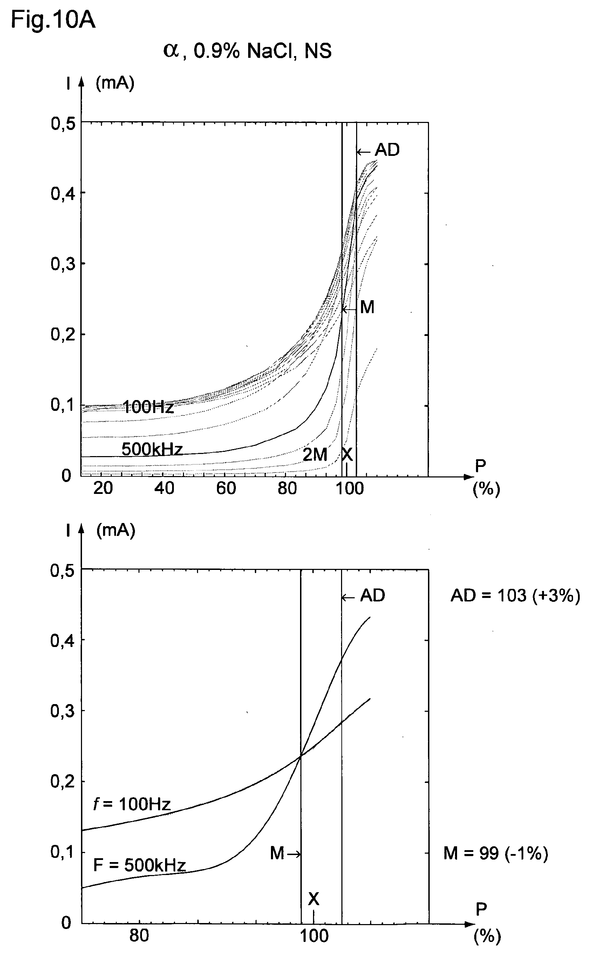

- les figures 10A à 13'C montrent une série de courbes de mesure d'amplitude en fonction de la profondeur d'insertion d'une électrode-sonde relevées expérimentalement sur les trois modèles α,β,γ de canaux radiculaires susvisés (Fig. 7, 8, 9), ces canaux étant irrigués successivement avec trois solutions aqueuses (indice de figure n°A : NaCl à 0,9%; indice B: NaClO à 2,5%; indice C : NaClO à 5%) avec diverses sondes et permettant de localiser la position de l'apex selon l'invention ;

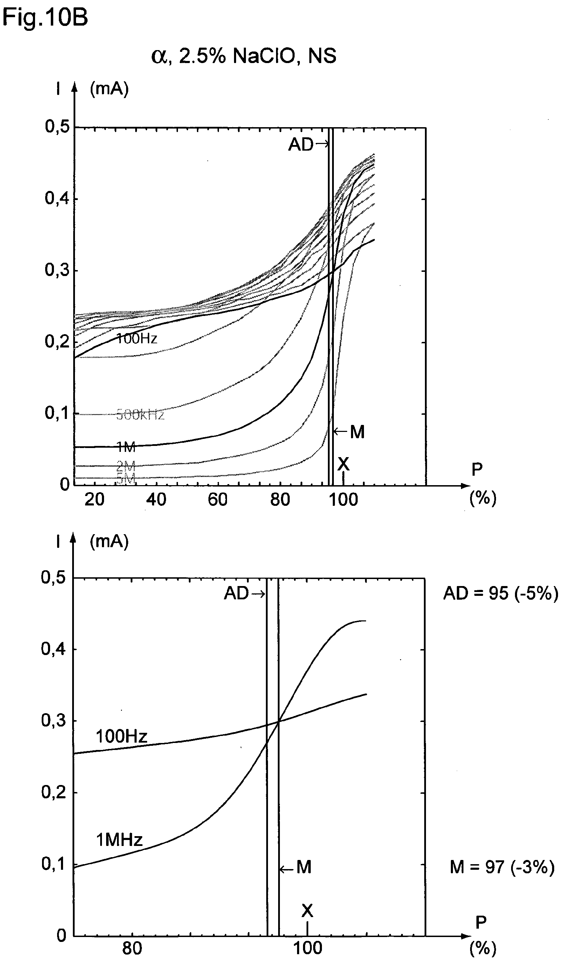

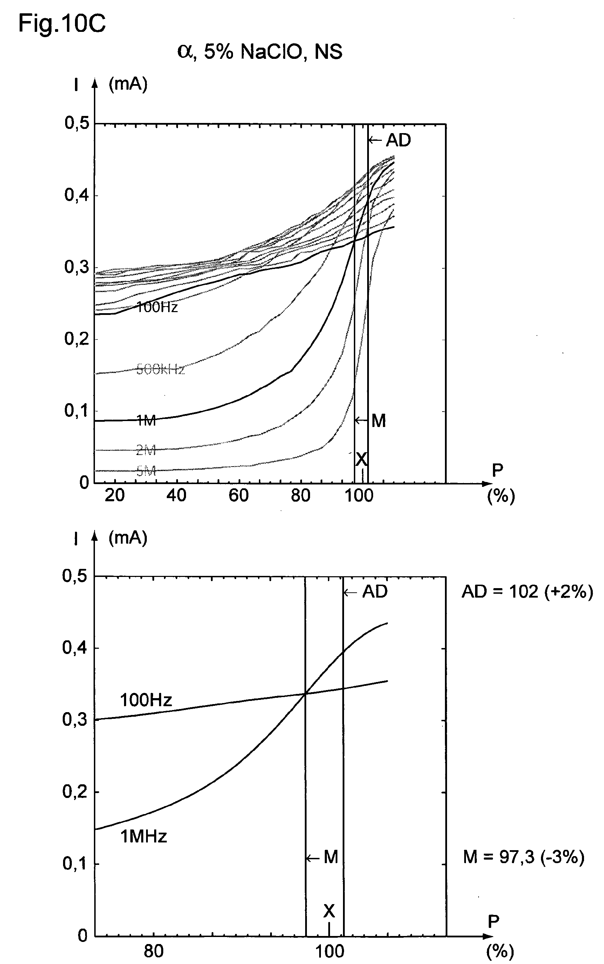

- les figures 10A, 10B, 10C montrent les courbes d'amplitude/profondeur relevées sur le modèle de canal radiculaire α de la figure 7 avec une sonde métallique nue et en présence, respectivement, de solution de NaCl à 0,9% (10A) de NaClO à 2,5% (10B) et de NaClO à 5% (10C);

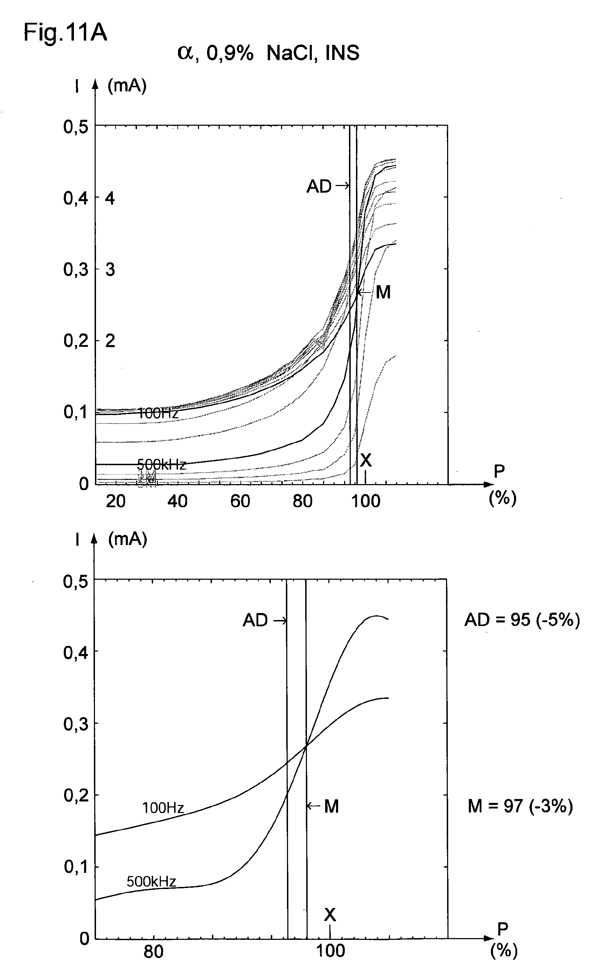

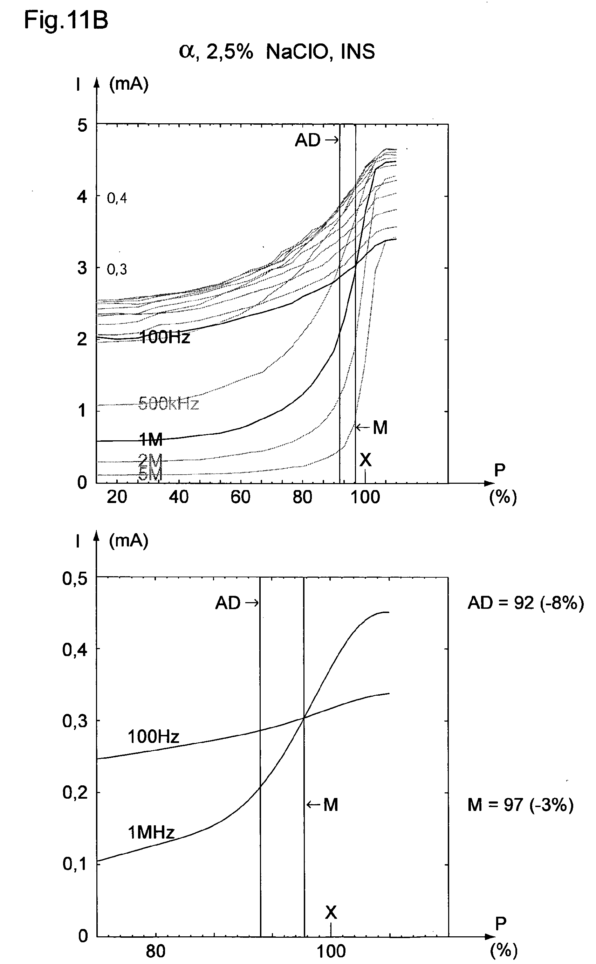

- les figures 11A et 11B montrent les courbes amplitude/profondeur relevées sur le modèle de canal radiculaire α de la figure 7 avec une sonde recouverte d'isolant en présence respectivement des mêmes solutions (Fig. 11A : 0,9% NaCl)(Fig. 11B : 2,5% NaClO) ;

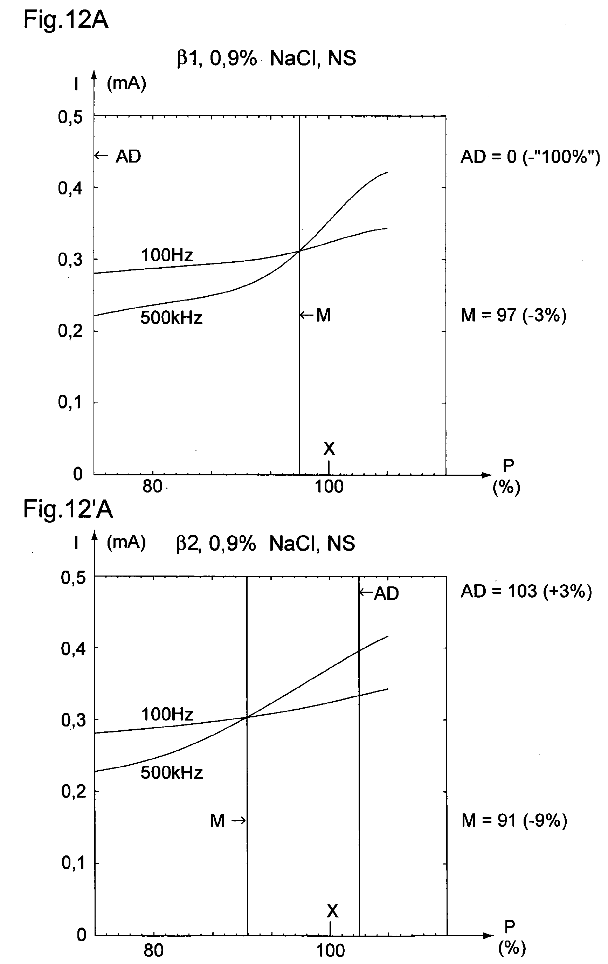

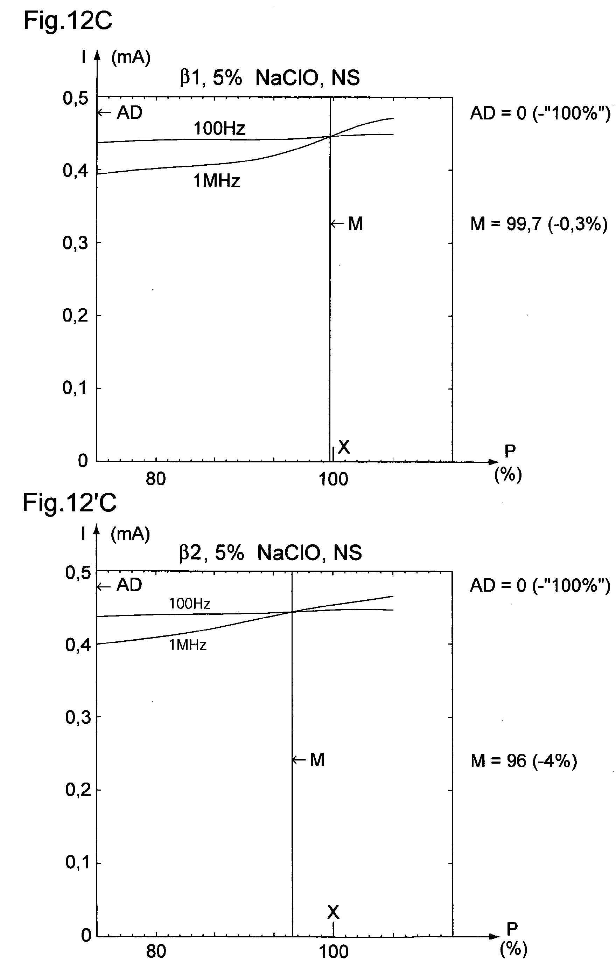

- les figures 12A-12'A et 12C-12'C montrent les courbes amplitude/profondeur relevées alternativement (12, 12') dans le premier et le second canal radiculaire β1 et β2 du modèle de racine dentaire β à embranchement fourchu de la figure 8 en présence respectivement des mêmes solutions (Fig.12A-12'A : 0,9% NaCl) (Fig. 12C-12'C : 5% NaClO) ;

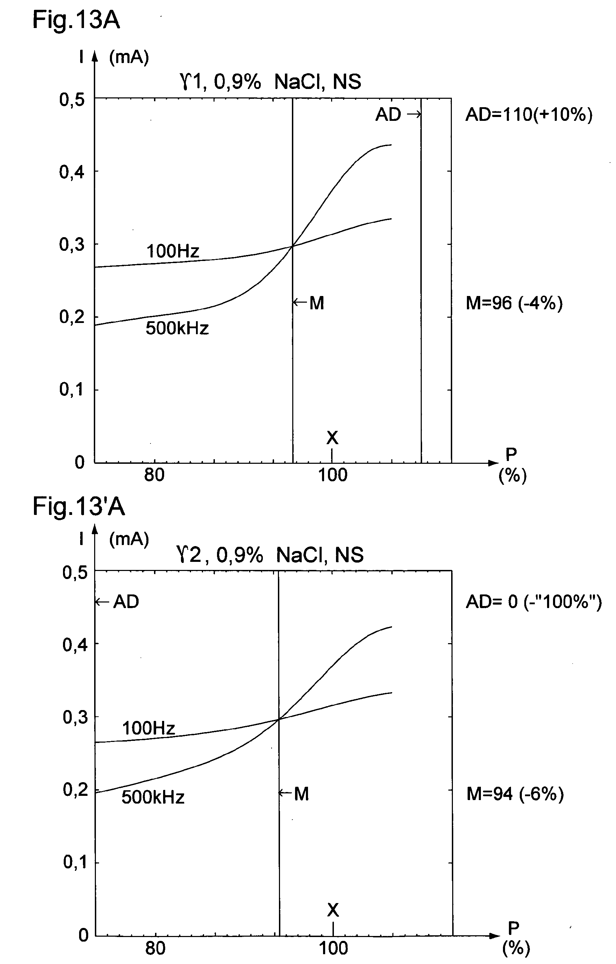

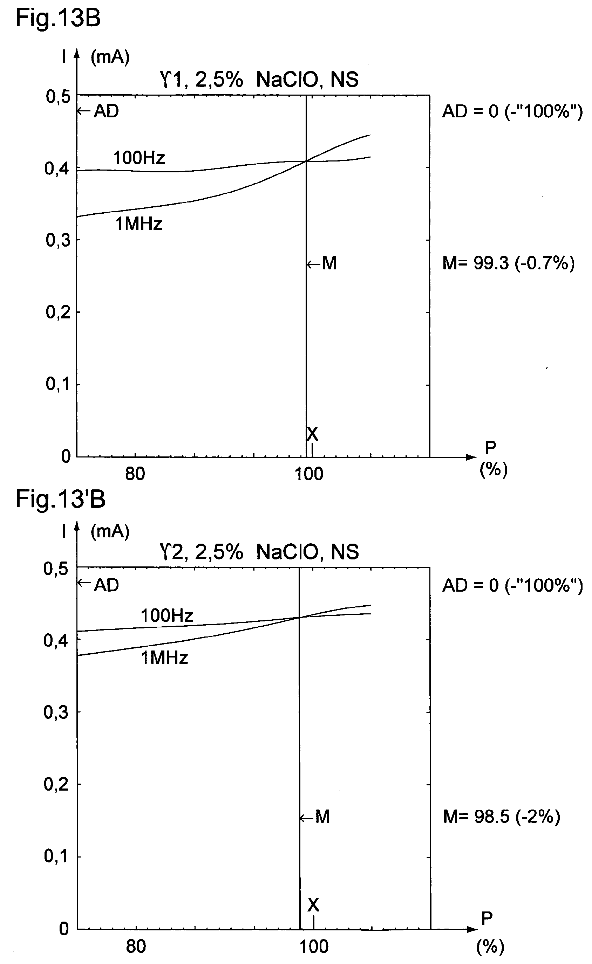

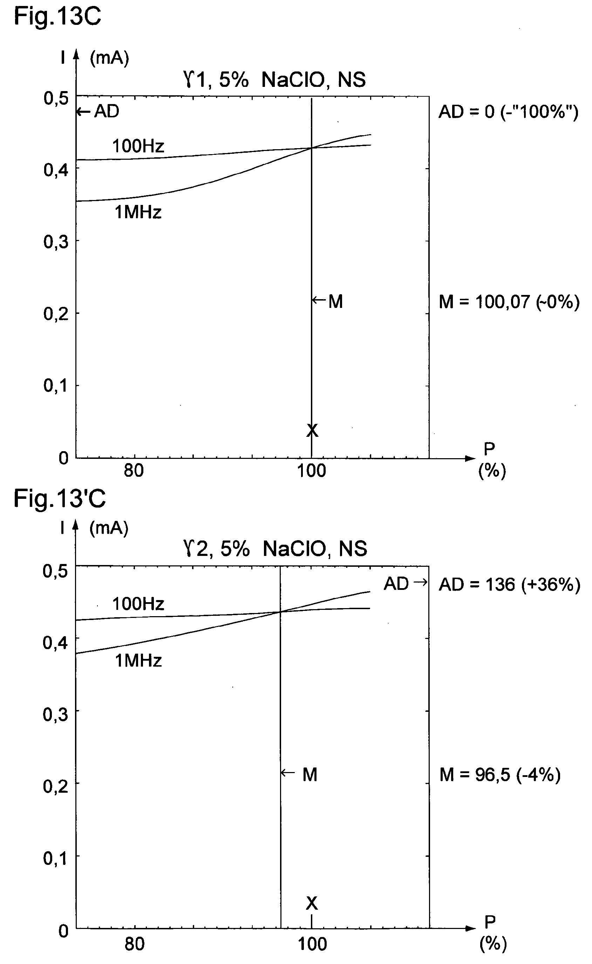

- les figures 13A-13'A, 13B-13'B et 13C-13'C montrent les courbes relevées alternativement (13/13') dans le premier et le second canal radiculaire γ1,γ2 du modèle de racine ramifiée γ de la figure 9 avec une sonde métallique nue en présence respectivement des mêmes solutions (Fig. 13A-13'A: 0,9% NaCl) (Fig. 13B-13'B : 2,5% NaClO) (Fig. 13C-13'C : 5% NaClO) ;

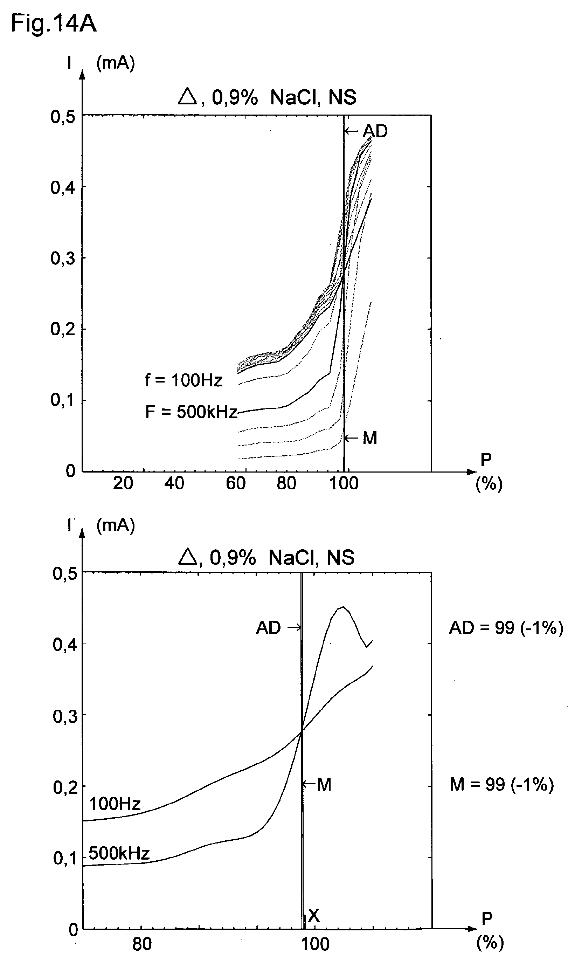

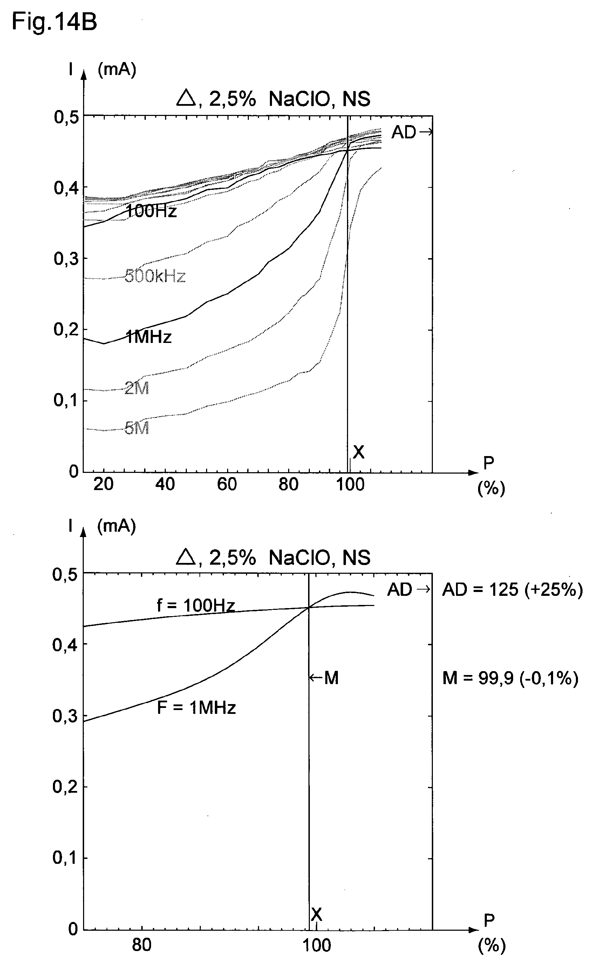

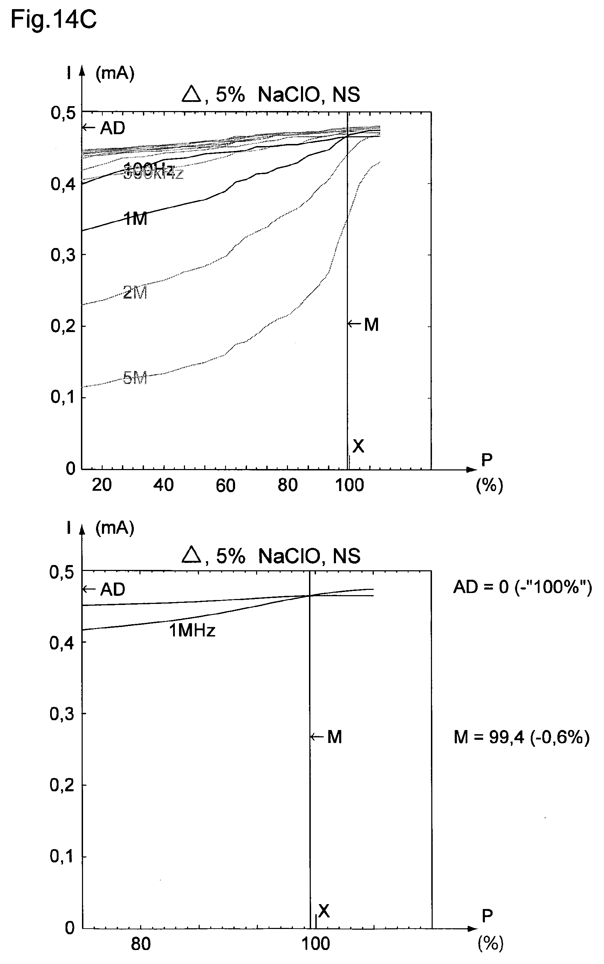

- les figures 14A, 14B et 14C donnent des courbes amplitude/profondeur relevées expérimentalement sur une **dent réelle Δ** à deux fréquences f=100Hz et F=0,5 ou 1 MHz, en présence respectivement des trois solutions irrigantes (Fig. 14A : 0,9% NaCl) (14B : 2,5% NaClO) (14C : 5% NaClO) ; et,

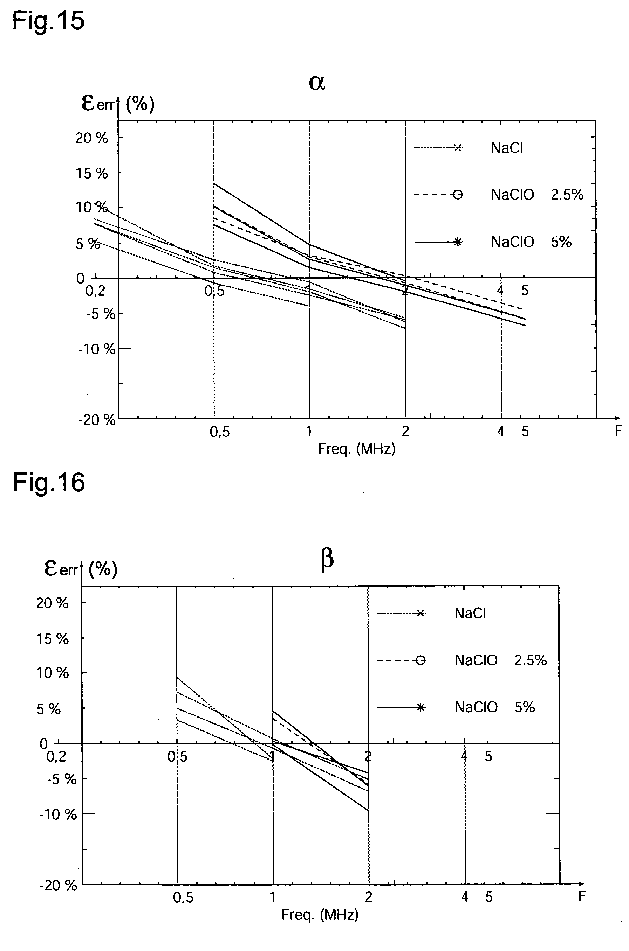

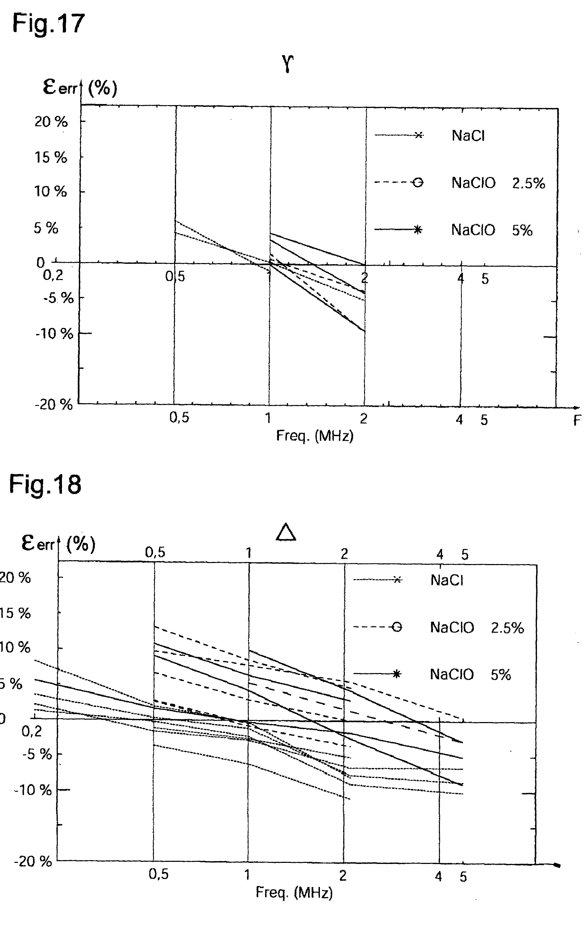

- les figures 15 à 18 sont des diagrammes représentant plusieurs nuages de points donnant des évaluations des erreurs de mesure εₑᵣᵣ en fonction de la valeur de la fréquence supérieure F choisie lors de campagnes de mesures de profondeur de canaux radiculaires α,β,γ ; le diagramme de la figure 15 regroupe les évaluations d'erreur de mesure ε selon la fréquence sup. F pour toute une série de mesures de profondeur du canal radiculaire du modèle dentaire α de la figure 7 ; les figures 16 et 17 regroupent les évaluations d'erreur ε en fonction de la fréquence supérieure F pour les modèles de canaux radiculaires β et γ des figures 8 et 9 respectivement ; la figure 18 regroupe les évaluations d'erreur ε selon F pour plusieurs séries de mesures de profondeur d'apex sur plusieurs spécimens de dents réelles.

Dans la présente, on met en oeuvre une sonde endodontique conductrice, qui peut revêtir différentes formes et notamment être formée par une tige, une pointe ou une lime (en anglais : "file") métallique qui joue le rôle d'électrode. De préférence, l'électrode-sonde se présente comme une étroite tige métallique, longiligne et flexible, de longueur centimétrique (de l'ordre d'un centimètre ou d'une fraction de centimètre à quelques centimètres, pas plus d'un décimètre, typiquement 2-3 cm). Cette tige ou lime, de section ronde ou autre, présente un diamètre (dimension transversale) nettement inférieur à sa longueur. La tige métallique, qui joue le rôle d'électrode terminale (en contact électrique avec une électrode de connexion intermédiaire et/ou reliée à une borne de sortie du générateur GF), peut être couverte sur tout ou partie de sa longueur par un revêtement isolant, comme dans les relevés de courbes expérimentales rapportées par la suite en regard des figures 11A et 11B. On peut notamment utiliser des sondes endodontiques disponibles dans les réseaux commerciaux, et les résultats d'expérimentation rapportée par la suite seront confrontés à ceux d'un dispositif de localisation d'apex existant dans l'état de la technique antérieur, les résultats obtenus avec ce dispositif localisateur d'apex disponible antérieurement étant reporté sous la référence AD sur les figures 10A et suivantes.

En se reportant à la figure 5A, par référence, il apparaît que le circuit électrique du dispositif de localisation d'apex, selon l'invention, met en oeuvre avantageusement un générateur fréquentiel GF de signaux alternatifs sinusoïdaux, relié en série avec une première électrode conductrice E1 qui est formée par la sonde endodontique S qui s'engage dans le canal radiculaire CR d'un specimen de dent (ci-après Δ) ou d'un modèle de canal dentaire (ci-après α,β,γ). Le circuit comporte une seconde électrode de masse E0 reliée en série avec un montage ou un appareillage de mesure AM d'intensité du courant alternatif qui traverse les deux électrodes E0-E1 et est produit par le générateur de signaux alternatif GF qui est ici un générateur à agilité de fréquences.

Le montage et l'appareillage de mesure AM doivent permettre de mesurer l'amplitude des signaux alternatifs sinusoïdaux et plus précisément l'amplitude de l'intensité des courants alternatifs. Cependant, selon l'exemple de montage de mesure de la figure 5A, l'appareil de mesure AM peut mesurer l'amplitude en tension des signaux alternatifs notamment l'amplitude absolue (en valeur crête, efficace ou RMS) et être branché en parallèle sur les bornes d'une résistance de référence de mesure Rm placée en série avec les électrodes E0-E1 et le générateur GF à agilité de fréquence du circuit. L'appareil de mesure AM peut être, selon un premier exemple expérimental 5B,5C,5D, un oscilloscope couvrant une gamme étendue de fréquences et dont les bornes de mesure sont connectées aux bornes d'une résistance de mesure Rm de valeur très faible vis-à-vis de l'impédance d'entrée Zi de l'appareil AM, c'est-à-dire vis-à-vis de sa résistance d'entrée Ri et surtout de la capacité Ci entre ses bornes d'entrée. A titre d'exemple, avec une résistance d'entrée de dix MégaOhms (Rᵢ = 10 MΩ) et une capacité de quinze picoFarad (Cᵢ = 15 pF), une résistance de mesure Rm ayant une valeur de l'ordre de dix mille ohms ou moins, par exemple un ou quelques milliers d'ohms, permet de disposer d'une fréquence de coupure élevée supérieure à un MégaHertz (fc>1MHz) et même à plusieurs MégaHertz, voire une dizaine de MégaHertz (fc>10 MHz).

De façon avantageuse, une telle impédance de mesure (*i*.*e*. Rₘ // Ci = 10kΩ // 15pF) se trouve être adaptée à l'impédance intrinsèque Z qui apparaît entre les deux électrodes E1 et E0, c'est-à-dire l'impédance Z propre au canal radiculaire CR.

Le circuit GF ou les moyens générateurs de signaux alternatifs doivent être aptes à produire des signaux de fréquences dans des gammes de fréquences éloignées et notamment pouvoir produire des signaux alternatifs à au moins deux fréquences (f, F) choisies dans des bandes de fréquences opposées, c'est-à-dire dans des décades de fréquences distinctes et de préférence distantes, i.e. séparées par une ou plusieurs décades ou bandes de fréquences. L'une des deux fréquences (f) est désignée dans la présente comme étant la première fréquence, basse fréquence ou fréquence inférieure ; l'autre fréquence (F) est appelée seconde fréquence, haute ou supérieure. La première fréquence f appartient à un domaine considéré usuellement comme le domaine des basses fréquences électriques, c'est-à-dire le domaine des fréquences inférieures aux très basses fréquences radioélectriques (f < 3 kHz - limite inférieure de la bande conventionnelle n°4 en Radioélectricité). La première fréquence f appartient notamment au domaine de fréquences incluant la bande conventionnellement numérotée 2 autour de 10² Hz (30Hz ≤ f ≤ 300Hz), tandis que la seconde fréquence F appartient à un autre domaine de fréquence opposé, considéré usuellement comme le domaine des hautes fréquences électriques, i.e. le domaine des radiofréquences (F » 3 kHz), domaine qui inclus notamment la bande des moyennes fréquences radioélectriques conventionnellement numérotée 6 autour de 10⁶ Hz (300 kHz≤F≤3MHz). Ladite bande de fréquences n°6 qui couvre une décade de fréquences autour d'environ 1 MHz est aussi connue en RadioElectricité, sous les appellations de Moyennes Fréquences, notée MF, de Petites Ondes (P.O.) ou Ondes Moyennes, en abréviation métrique B.hm ou O.hm pour bande ou ondes hectométriques.

Consécutivement, l'appareillage de mesure doit couvrir une plage de fréquence étendue couvrant des gammes de fréquences englobant les bandes allant de fréquences inférieures aux très basses fréquences radio ou basses fréquences électriques (f = 30 à 300 Hz ou 3000 Hz) et les bandes des moyennes fréquences radio ou hautes fréquences (F = 300 kHz à 3MHz, voire plus).

Dans l'exemple de circuit de mesure de la figure 5A, l'appareil AM mesure une valeur absolue RMS de l'amplitude de la tension U aux bornes de cette résistance de mesure Rm.

De telles mesures correspondent à des mesures de l'amplitude absolue (RMS) de l'intensité I du courant qui traverse le canal entre les électrodes E0-E1 et tout le circuit excité par le générateur de fréquence GF, qui produit une tension de signal alternatif à la fréquence déterminée sélectionnée par sa commande de sélection de fréquence.

Par valeur absolue d'amplitude, il peut s'agir indifféremment d'une valeur crête du signal alternatif Imax, d'une valeur crête à crête 2.Imax, d'une valeur efficace calculée (i.e. Imax/√2), d'une valeur efficace vraie leff, d'une valeur dite RMS (abréviation de l'anglais "Root Mean Square"pour Racine de la Moyenne des Carrés des valeurs instantanée d'amplitude) ou d'autres mesures de valeurs absolues, notamment de valeurs proportionnelles ou liées aux valeurs absolues précitées, du moment qu'il s'agit de valeurs normées donnant une norme de l'amplitude du signal alternatif.

Plusieurs séries de campagne de mesures séquentielles approfondies ont été menées par les Inventeurs en couvrant des gammes de fréquences allant de 100Hz à 5MHz, en enregistrant successivement des séquences de courbes de mesure à des fréquences établies espacées par saut de fréquence exponentiel (i.e. séries 1,2,5,10,...,100,200,500,1000,...,500000,1000000, 2.10⁶, 5.10⁶...Hz) en se concentrant en particulier sur l'allure des courbes d'amplitude des signaux alternatifs relevées à ces diverses valeurs de fréquences en fonction de la distance de l'extrémité de la sonde endodontique par rapport à l'extrémité du canal radiculaire obtenue en faisant varier la profondeur d'insertion de la sonde à l'intérieur du canal radiculaire d'un grand nombre de modèles de canaux dentaires et de dents réelles.

La figure 6A montre l'allure générale d'une série de courbes de mesures obtenues ainsi en mettant en oeuvre un tel système AM de mesure d'amplitude des signaux alternatifs produits par un générateur GF à agilité de fréquences dans un circuit-série comportant les deux électrodes conductrices E0-E1, l'électrode-sonde endodontique E1-S étant engagée dans un canal radiculaire CR (modèle de canal dentaire ou specimen de dent réelle). Chaque courbe de mesure est relevée à une fréquence déterminée. La courbe rassemble un ensemble de points de mesure à ladite fréquence, en fonction de la profondeur d'insertion atteinte par l'extrémité de la sonde. Les points de mesure sont relevés en enfonçant la sonde pas à pas en profondeur STP dans le canal radiculaire. L'insertion de la sonde s'effectue sur un banc micromécanique à déplacement micrométrique dont le pas et la précision de positionnement obtenus sont nettement en deçà du millimètre et de l'ordre d'une centaine de micromètres ou moins. A chaque pas STP, on mesure l'amplitude absolue (RMS) du signal alternatif à la fréquence sélectionnée, aux bornes de la résistance de mesure Rm.

Chaque courbe de la figure 6A rassemble ces points de mesure de niveau d'amplitude absolue de l'intensité I (I=U/Rm) en fonction de la profondeur STP ou P d'insertion de l'extrémité de l'électrode conductrice E1 formée par la sonde endodontique S dans le canal radiculaire CR, la sonde étant déplacée pas à pas STP par le micromécanisme.

Comme on peut le voir sur chaque courbe de la figure 6A et des figures suivantes 6B et 10A-14C, il apparaît que chaque ensemble de plus d'une centaine de points de mesures établis à une fréquence déterminée forme un ensemble de mesures tout à fait cohérent et continu, formant une courbe lisse, régulière, sans discontinuité.

De façon générale, d'après l'allure d'ensemble des courbes de mesure d'amplitude des figures 6A-6B et suivantes 10A-14C, on observe que chaque courbe comporte au moins trois portions I, II, III,... comme suit :
- une première portion I, dans laquelle le niveau d'amplitude des signaux alternatifs est stable à un niveau plancher ou croît très légèrement avec la profondeur d'insertion P de la sonde ; cette première portion I correspond à la phase initiale d'insertion de la sonde au début du canal dentaire et s'étend à toute la phase où l'extrémité de la sonde parcourt la majeure partie de la longueur du canal radiculaire, i.e. depuis l'introduction de la sonde dans la couronne dentaire (partie coronaire) jusqu'à la zone apicale II ;
- une seconde portion II, dans laquelle le niveau d'amplitude (RMS) des signaux croît rapidement en fonction de la profondeur d'insertion de la sonde ; comme indiqué par un trait d'axe vertical APX sur la figure 6A ou une côte X de profondeur P sur les figures suivantes 6B et 10A-14C, cette seconde portion II correspond à la phase dans laquelle l'extrémité de la sonde traverse la zone apicale autour de la position de l'apex APX ;
- une troisième portion III, le cas échéant, dans laquelle le niveau d'amplitude des signaux alternatifs croît moins rapidement et/ou ne croît plus avec la profondeur P d'insertion de la sonde, si bien que le niveau d'amplitude se rétablit et se stabilise éventuellement à un niveau plafond terminal ; cette dernière portion III correspond à une phase où l'extrémité de la sonde a dépassé la zone apicale et s'enfonce en profondeur au delà de la position X de l'apex APX ; le niveau de signal varie peu ou ne varie plus par rapport au niveau plafond terminal.

Comme le montre la figure 6A, sur les courbes relevées aux basses fréquences BF, i.e. aux premières valeurs inférieures de fréquences de par exemple 100 Hz, 200 Hz, 500 Hz,..., 10 kHz, 20 kHz, 50 kHz etc., on observe que chaque courbe de niveau d'amplitude croît modérément, entre un niveau plancher initial assez élevé, aux environs de I=0,15 à 0,2 mA, pour atteindre un niveau plafond final s'élevant entre I=0,3 et 0,45 mA.

On distingue en fait plusieurs faisceaux de courbes, notamment un premier faisceau BF de courbes relevées à Basse Fréquence (i.e. 100Hz, 200Hz, 500Hz, ...,10 kHz, 20 kHz, 50 kHz) et un autre faisceau HF regroupant des courbes relevées à Haute Fréquence (i.e....200 kHz, 500 kHz, 1 Mhz, 2 MHz, 5 MHz, ...). Parmi le premier faisceau BF de courbes d'amplitude en Basse Fréquence, la courbe établie à la première valeur de fréquence inférieure f=100 Hz paraît avoir la plus faible variation de niveau d'amplitude entre un niveau plancher initial moyen I1 ≈ 0,15 mA et un niveau plafond terminal I3 ≈ 0,325 mA. Dans l'autre faisceau HF de courbes d'amplitude en Haute Fréquence HF, les courbes s'étagent régulièrement par niveaux décroissants avec la croissance de leur fréquence de mesure F. La courbe établie à la plus grande valeur de fréquence supérieure F=5MHz affiche à la fois un niveau initial plancher mimimum et un niveau terminal plafond minimal. Parmi ce faisceau de courbes Haute Fréquence HF, les courbes établies aux valeurs intermédiaires de fréquence haute F = 500 kHz, 1 MHz, 2 MHz apparaissent comme celles connaissant la plus grande variation de niveau d'amplitude, entre un niveau initial plancher particulièrement faible et un niveau terminal plafond particulièrement élevé.

Cependant, comme le suggèrent les figures 6A-6B et les figures 10A-14C, les niveaux de ces courbes sont fluctuants et varient en fonction de divers paramètres, comme le type de spécimen dentaire, la configuration géométrique du ou des canaux radiculaires (large, étroit, ramifications et/ou aberrations), la présence de milieux électrolytiques (liquides physiologiques, solutés ioniques dilués à base de NaCl ou de NaClO) et surtout, de la fréquence et de choix de fréquences particulières d'après l'invention.

Au cours de ces campagnes de mesure, les Inventeurs se sont concentrés sur des relevés cumulatifs de courbes synthétiques de mesure d'amplitude, les mesures portant sur l'amplitude absolue de l'intensité I des signaux alternatifs tel que mesuré aux bornes de la résistance de mesure Rm en série avec les électrodes E0-E1, les courbes étant établies en fonction de la profondeur d'insertion de l'électrode-sonde endodontique S et selon la valeur de chacune des fréquences sélectionnées. Dans ces campagnes d'études systématiques, les inventeurs ont mis en oeuvre un générateur GF de signaux alternatifs à agilité de fréquences couvrant des bandes fréquentielles allant de la bande conventionnelle numéro deux (correspondant à la bande autour de 10² Hz soit 30 à 300 Hertz), jusqu'aux bandes conventionnelles numéro six et sept (correspondant à la bande autour de 10⁶ Hz, soit 300 kHz à 3MHz et à la bande des 10⁷ Hz, soit 3MHz à 30MHz).

Les Inventeurs ont sélectionné parmi toutes ces séries de courbes, certaines des courbes établies à des fréquences déterminées situées aux extrémités du spectre fréquentiel des gammes de fréquences étendues couvertes par le générateur, et se sont aperçus que ces courbes sélectionnées établies à des fréquences particulières opposées ont la particularité de se croiser.

Plus précisément le diagramme de la figure 6B montre une sélection de certaines courbes particulières de la figure 6A, les deux courbes sélectionnées étant relevées à deux fréquences déterminées f et F appartenant aux bandes fréquentielles opposées aux extrémités du spectre fréquentiel du générateur de signaux alternatifs GF. Les deux courbes f et F du diagramme 6B donnent l'allure de l'évolution des niveaux d'amplitude absolue RMS (en intensité I ou en tension V = Rm . I) en fonction de la profondeur P de l'extrémité de la sonde S dans le canal d'une racine dentaire CR.

Dans le détail, sur le diagramme de courbes de la figure 6B, on voit que chaque courbe comporte plusieurs zones I,II,III,... correspondant aux deux ou trois portions du canal dentaire, dans lesquelles les niveaux d'amplitude des signaux sont hiérarchisés, comme suit :
- dans une première zone I, correspondant au début de l'introduction de la sonde au niveau de la partie coronaire de la dent (couronne) puis à l'engagement et au parcours de la majeure partie de la longueur du canal radiculaire, les niveaux d'amplitudes I1 et I2 relevées aux deux fréquences f = 100 Hz et F = 500 kHz, varient peu en fonction de la profondeur (la profondeur P varie ici entre 0 et 1 à 3 centimètres, la distance D par rapport à l'apex X variant de manière complémentaire entre 10-20 millimètres et 0mm). Dans cette première zone I, le premier niveau d'amplitude I1 relevé à la première fréquence inférieure (f= 100 Hz) est nettement supérieur au second niveau d'amplitude I2 de l'intensité I ou de la tension U du signal alternatif relevé à la seconde fréquence supérieure (F = 500 kHz) ;
- dans une seconde zone II, lorsque l'extrémité de la sonde atteint des profondeurs d'insertion P entre 10 et 20 mm, correspondant à la partie terminale du canal radiculaire CR et à l'approche de l'apex APX (positionné à la cote P=100% *i.e.* D=0), les deux niveaux If et IF d'amplitudes absolues (RMS I=V/Rₘ) des signaux alternatifs relevés à ces deux fréquences, la fréquence basse f et la fréquence haute F (ici f=100 Hz et F = 500 kHz), augmentent rapidement avec l'augmentation de la profondeur P d'insertion de la sonde S et la diminution de la distance D par rapport à l'apex APX, X.
- Plus précisément dans cette seconde zone II, correspondant à la fin du canal radiculaire CR, le premier niveau d'amplitude If établi à la première fréquence basse (f = 100 Hz) présente un taux de croissance (I3-I1/ΔP) positif en fonction de la profondeur d'insertion P de l'électrode-sonde mais inférieur au taux de croissance (I4-I2/ΔP) du second niveau d'amplitude IF relevé à la seconde fréquence haute (F = 500 kHz). A tel point que le second niveau IF établi à la seconde fréquence haute (F = 500 kHz) se rapproche, rattrape et rejoint jusqu'à égaliser sensiblement et croiser C le premier niveau If établi à la première fréquence basse (f = 100 Hz).
- Par la suite, après ce point de coïncidence ou de croisement C, dans une troisième zone III, le second niveau IF d'amplitude RMS du signal alternatif établi à la seconde fréquence haute (F = 500 kHz) continue à croître avec un taux de croissance (ΔIF/ΔP) supérieur ou du moins sensiblement égal à celui (ΔIf/ΔP) du premier niveau If d'amplitude de signal relevé à la première fréquence basse (f = 100 Hz).

Ainsi, au-delà de ce point C de jonction et/ou de croisement des deux niveaux If et IF, dans la dernière zone, le second niveau d'amplitude IF relevé à la seconde fréquence (F = 500 kHz) continue de croître avec un taux de croissance supérieur et à s'écarter par rapport au premier niveau d'amplitude If relevé à la première fréquence basse (f = 100 Hz) ou maintient l'écart avec un taux de croissance sensiblement égal au premier niveau d'amplitude If avant qu'ils se stabilisent à des niveaux IF=I4 et If=I3 qui ne varient guère.

Il se trouve que, de façon inattendue, selon l'étude des Inventeurs, le point C où se rejoignent et/ou se croisent les deux courbes de niveaux If et IF d'amplitude absolue des signaux (I ou V/Rm), établies ici à f = 100 Hz et F = 500 kHz, se situe à une profondeur M qui correspond apparemment à la profondeur X de l'apex APX (P=100%).

Il reste que l'objectif important lors des opérations de chirurgie dentaire, telle qu'une opération de nettoyage et de mise en forme du canal endodontique, et le but fonctionnel même des systèmes de localisation d'apex est de ne pas dépasser l'apex APX et d'éviter de s'engager au-delà du *foramen apical* FA, mais de s'en approcher au plus près en restant en deçà du *terminus apical* APX. Dès lors, selon l'invention, il est prévu simplement de détecter un tel point de coïncidence C où se rejoignent et s'égalisent les deux niveaux If et IF d'amplitude des signaux alternatifs relevés à des fréquences opposées f et F choisies dans les bandes TBF/BF et MF/HF du spectre fréquentiel du circuit générateur de fréquences GF. Ainsi, grâce à l'invention, on peut avantageusement ne pas dépasser ledit point C et éviter de s'engager dans la troisième zone III dans laquelle la hiérarchie du premier niveau If et du second niveau IF s'inverse et où le second niveau IF->I4 devient supérieur et s'écarte de plus en plus par rapport au premier niveau If->I3 (divergence du second niveau IF au-dessus du premier niveau If d'amplitude RMS aux profondeurs P>100% au delà de la position X de l'apex APX).

L'avantage premier de l'invention est que la détection d'un tel point de coïncidence C constitue à lui seul un critère absolu de mesure de la position de l'apex. La détection de ce point de coïncidence C ne se réfère pas à un seuil et ne nécessite pas de régler ou d'étalonner un seuil de référence relatif. La détection du point de coïncidence C selon l'invention permet avantageusement une détermination absolue de la position de l'apex.

Grâce au dispositif de l'invention, il est inutile de chercher à confirmer que les deux niveaux se croisent bien en recherchant la troisième zone III dans laquelle le second niveau IF devient supérieur au premier If et/où ils s'écartent à nouveau (zone III où If→I3 et IF→I4 redeviennent nettement distant). Il suffit simplement de détecter le point C où les deux niveaux If et IF se rejoignent et s'égalisent sensiblement pour repérer l'apex APX, en évitant de dépasser ce point de coïncidence C, par mesure de précaution.

Ainsi, dans son principe, le dispositif de l'invention permet de mettre en oeuvre un procédé de localisation d'apex à l'extrémité de la racine ou d'une des racines d'une dent, procédé destiné plus précisément à localiser la constriction apicale au fond de chaque canal radiculaire de la dent et à déterminer la position en profondeur du terminus apical à l'extrémité du canal radiculaire, le procédé de localisation d'apex mettant en oeuvre un dispositif comprenant une première électrode-sonde endodontique conductrice apte à être insérée dans le canal radiculaire de la dent, une seconde électrode conductrice conformée pour être mise en contact conducteur électriquement avec une muqueuse buccale, un circuit ou des moyens générateurs de fréquences aptes à produire des signaux électriques alternatifs à plusieurs fréquences (au moins deux fréquences : une fréquence inférieure, basse fréquence et une fréquence supérieure, haute fréquence), et des moyens de mesure de grandeur des signaux électriques alternatifs produits dans un circuit électrique comprenant lesdits moyens générateurs de fréquences, la première électrode insérée dans le canal radiculaire, la seconde électrode au contact de la muqueuse buccale et les moyens de mesure, le procédé comportant des étapes consistant à :
- exciter le circuit et mesurer le niveau de grandeur des signaux électriques alternatifs dans le circuit, respectivement, à une fréquence inférieure f et à une fréquence supérieure F (sélectionnées de sorte que le premier niveau If établi à la fréquence inférieure f (basse fréquence) soit initialement (If=I1) supérieur au second niveau IF=I2 établi à la fréquence supérieure F (haute fréquence)) ;
- détecter un point de coïncidence C où les deux niveaux If et IF respectifs de la grandeur électrique (Amp. RMS I) mesurée aux dites fréquences inférieure f et supérieure F se rejoignent et se croisent, lesdites fréquences inférieure et supérieure étant suffisamment éloignées pour qu'un tel point de coïncidence C existe, ledit point C/M concordant avec la position X de l'apex.

L'existence de ce point de coïncidence C qui permet à première vue, de caractériser une mesure de profondeur de la position X de la constriction apicale, selon l'étude préliminaire des Inventeurs, se devait d'être approfondie par une campagne d'étude plus approfondie s'étendant à divers modèles de canaux radiculaires et une série de spécimens de dents réelles.

Une campagne extensive de mesures expérimentales systématiques de courbes de niveau d'amplitude absolu d'intensité de signal alternatif en fonction de la profondeur d'insertion d'électrode-sonde et des fréquences choisies a été menée pour affiner les résultats. Cette campagne d'étude systématique a été effectuée avec des sondes adaptatives en comparant les résultats M, obtenus selon l'invention, par rapport à des résultats de référence AD obtenus avec un dispositif de localisation d'apex à sonde endodontique existant antérieurement dans l'état de la technique et disponible dans le commerce. Ces résultats de référence sont côtés sur les diagrammes de relevés de mesures sous la référence AD. Dans un premier volet de cette campagne extensive, les relevés expérimentaux ont été effectués sur des modèles de canaux de racines dentaires, dont trois échantillons α, β et γ sont représentés sur les figures 7 à 9.

Comme représenté sur la figure 7, le premier modèle de canal de racine dentaire (α) comporte une ouverture creusée en forme d'entonnoir se prolongeant par un canal étroit de diamètre sensiblement constant qui s'achève brusquement par un orifice débouchant (décrochements d'épaulements).
La figure 8 montre un autre modèle de canal de racine dentaire (β) présentant une bifurcation, la racine se subdivisant à sa partie terminale en deux canaux radiculaires β1,β2 et présentant chacun un orifice apical, de telles ramifications complexes existant dans la structure de bons nombres de dents (molaires à racines multiples, prémolaires à racines jumelles etc.).
La figure 9 montre un dernier modèle expérimental de canal de racine dentaire (γ) présentant des aberrations et bifurcations avec une ramification en deux canaux radiculaires γ1, γ2 et une excroissance latérale, ce type d'aberrations existant dans des racines dentaires réelles et étant particulièrement difficile à résoudre, c'est-à-dire à reconnaître, à discriminer et à distinguer avec les dispositifs à sonde endodontique de l'art antérieur.

D'autres résultats expérimentaux relevés sur des dents réelles seront détaillés par la suite dans la description ci-après.

Les figures 10A à 13'C montrent une série de résultats de mesure de profondeur de l'apex dans chacun des canaux radiculaires des modèles dentaires des figures 7, 8 et 9, ces résultats étant comparés à ceux obtenus avec un dispositif de localisation d'apex de l'état de la technique antérieur, disponible dans le commerce, ces résultats comparatifs étant indiqués par un trait de côte de référence AD.

Pour chaque figure 10, 11, 12-12' et 13-13' les diagrammes indicés A donnent les courbes amplitude/profondeur relevées en présence d'une solution irrigante de chlorure de sodium à près de 1% (solution aqueuse NaCl 0,9%) analogue au "sérum physiologique" et aux fluides organiques tels que le sang, la lymphe, la salive ou les fluides chargés de débris organiques.

Les diagrammes indicés B et C donnent respectivement des courbes relevées dans les mêmes conditions mais en présence de solutions de rinçage à base d'hypochlorite de sodium en concentration de 2,5% et 5% (10B, 11B, 13B-13'B : NaClO 2,5%) (10C, 12C-12'C, 13C-13'C : NaClO 5%).

Les solutions ioniques alcalines à base d'hypochlorite de sodium B et C (NaClO à 2,5% et 5%) sont fortement conductrices. L'étude porte en particulier sur l'influence de ces solutions électrolytique sur les résultats de mesure de profondeur, leur cohérence et le choix des fréquences de mesure, afin de vérifier si les mesures de profondeur concordent avec la position de l'apex, les dispositifs de localisation d'apex de l'art antérieur (AD) existant ayant le gros inconvénient d'être inopérants dans de telles conditions de présence de solutions fortement conductrices, notamment à base de NaClO qui est une solution désinfectante (liqueur de Dakin, analogue à la véritable Eau de Javel, nécessaire lors des opérations dentaires).

Les figures 10A, 11A, 12A, 12A-12'A et 13A-13'A, (en présence de solution saline commune de NaCl à 0,9%), représentent les premiers résultats de courbes d'amplitude des signaux, en fonction de la profondeur P de l'extrémité de la sonde ou de la distance D par rapport à la position connue de l'extrémité du canal (profondeur P côtée 100, distance D=0) pour les trois modèles α, β et γ. Comme l'indiquent les figures 10, 11, 12 et 13, il apparaît que le procédé selon l'invention permet d'obtenir d'excellents résultats de détermination de la profondeur P de l'apex en sélectionnant deux niveaux d'amplitudes If et IF relevés à deux fréquences particulières, la première à fréquence basse, i.e. f = 100 Hz, et la seconde à fréquence haute, i.e. F = 0,5 MHz. Les deux niveaux d'amplitude absolue RMS de l'intensité I des signaux fréquentiels se croisent en un point dont la position M correspond très précisément à la position réelle X de l'extrémité du canal radiculaire sur les premiers modèles dentaires à canal simple (fig. 10A-11B).

On constate sur les figures 10A à 11B, que la précision de la position M de l'apex déterminée selon l'invention, est mesurée avec une erreur minime, en deçà de -2% ou -3%, ce qui correspond à une meilleure résolution comparée à la marge d'erreur allant de -8% à + 3% donnée par le dispositif antérieur AD disponible dans le commerce.

En outre, surtout, le relevé du point de coïncidence des niveaux d'amplitude des signaux alternatifs relevés aux deux fréquences f = 100 Hz et F = 0,5 MHz indique une mesure M de profondeur d'apex qui se situe très légèrement en retrait (M<100%) avant d'atteindre la position réelle X de l'extrémité du canal radiculaire (erreur négative de -1% à -3%), tandis que la mesure de position AD indiquée par le dispositif antérieur existant dans le commerce est située, tantôt en deçà (AD«100%), tantôt au-delà (AD>100%) de la position réelle X de l'extrémité du canal radiculaire, ce qui signifie que l'opération dentaire peut dépasser la position de l'apex, ce que dentistes et patients cherchent absolument à éviter.

De façon avantageuse, dans le procédé de détection de point de coïncidence, le choix d'un couple de fréquences (f,F) adapté à la présence de liquide physiologique, tel qu'un première fréquence basse f dans la bande n°2 autour de 10² Hz appariée avec une seconde fréquence haute F dans la bande n°6, entre 0,3.10⁶ Hz et 3.10⁶ Hz, et plus précisément dans l'octave 0,5 MHz-1MHz ou l'octave supérieure et de préférence à une valeur de fréquence F d'environ un demi à un MégaHertz, permet d'obtenir d'excellent résultats de détermination M de profondeur d'apex et surtout d'obtenir une profondeur légèrement minorée qui permet d'éviter de dépasser la position réelle X de l'apex.

Les figures 10B, 10C et 11B concernent des courbes de niveaux d'amplitudes relevés sur le modèle de dent à canal simple α en présence de solution ionique alcaline à base de NaClO à 2.5%, à 5% et 2.5% respectivement.

La variation du taux de concentration de la solution d'hypochlorite de sodium entre 2,5% et 5% ne paraît pas modifier beaucoup les résultats obtenus avec le procédé de détection de point de coïncidence des niveaux selon l'invention.

Il apparaît qu'en cas de présence d'hypochlorite de sodium, sur les diagrammes 10B, 10 C et 11B d'amplitude, au début de l'introduction de la sonde aux faibles profondeurs d'insertion (0 < P « 100), les niveaux d'amplitude absolue (RMS) de l'intensité I des signaux alternatifs sont nettement plus élevés que les niveaux des courbes des figures 10A et 11A, en présence de solution saline à 0,9% NaCl, du fait que les solutions de NaClO sont plus fortement conductrices.

De ce fait, il apparaît préférable de sélectionner un autre choix de couple de fréquences basse et haute (f, F) pour détecter la position de l'apex avec la meilleure précision en présence de NaClO.

En cas de présence de solution d'hypochlorite de sodium, les figures 10B-10C et 11B indiquent que c'est le croisement des niveaux d'amplitude des signaux établis aux environs des deux fréquences basses f = 100 Hz et haute F = 1 MHz ou 2 MHz qui donne les meilleurs résultats de mesure M de position de l'apex X.

Au vu des résultats des figures 10B, 10C et 11B, il apparaît que le procédé de mesure de la position en profondeur de l'apex, selon l'invention consistant à détecter le point de coïncidence des deux niveaux établis à basse fréquence f et haute fréquence F, en particulier à f = 100 Hz et F = 1 MHz ou 2MHz environ, indiquent une position en profondeur M nettement plus précise que les résultats de mesure de profondeur AD établi avec le dispositif de localisation d'apex disponible antérieurement. Surtout, les mesures M de position en profondeur obtenues d'après l'invention ne sont pas majorées par rapport à la position X réelle de l'apex (M<X=100%).

Selon les résultats des figures 10B-10C-11B, en présence de solutions ioniques fortement conductrices, comme les solutions à base de NaClO à 2,5% ou 5%, le choix d'un couple de fréquences (f, F) adapté à de telles solutions, tel qu'une première fréquence basse f dans la bande n°2 autour de 10² Hz appariée avec une seconde fréquence haute F dans la bande n°6, autour de 10⁶ Hz, et plus précisément dans l'octave [1 MHz-2 MHz] ou une octave supérieure et de préférence avec une valeur de fréquence F d'environ un ou deux MégaHertz, permet d'obtenir d'excellent résultats de détermination de profondeur d'apex et surtout d'obtenir une profondeur M légèrement minorée qui permet d'éviter de dépasser la position réelle X de l'apex.

Les figures 12C-12'C et 13B-13'B, 13C-13'C donnent les courbes de niveaux d'amplitude et les mesures M de position en profondeur relevées en présence d'hypochlorite de sodium (indices B : 2,5% NaClO et C : 5% NaClO), pour un tel choix de fréquences déterminées sur les modèles de dent β et γ présentant des canaux radiculaires complexes, fourchus ou ramifiés avec aberration.

Les diagrammes 12A et 12C correspondent aux mesures de profondeur d'apex sur le premier canal β1 du modèle dentaire β de la figure 8.

Les diagrammes 12'A et 12'C correspondent aux mesures de profondeur de l'autre apex sur le second canal β2 du modèle β de la figure 8.

Les diagrammes 13A, 13B et 13C correspondent aux mesures de profondeur de l'apex sur le premier canal radiculaire γ1 du dernier modèle γ de canal dentaire illustré sur la figure 9.

Les diagrammes 13'A, 13'B et 13'C correspondent aux mesures de profondeur de l'autre apex sur l'autre canal de racine γ2 du modèle γ de la figure 9.

Il apparaît que le dispositif de localisation d'apex disponible antérieurement AD ne donne aucun résultat de mesure (AD=0 soit une "erreur de -100%") sur ces modèles complexes de canaux radiculaires présentant des aberrations ou des bifurcations de racines. Le dispositif antérieur AD ne donne aucune indication de profondeur des extrémités des canaux radiculaires, ni sur l'un, ni sur l'autre des canaux radiculaires ramifiés. Le dispositif ne fournit pas de résultat exploitable, ni en présence de solution saline de NaCl (figure 12A : β1, 0.9% NaCl, aucune indication de mesure ; figure 12'A : β2, 0.9% NaCl : indication de profondeur surestimée à éviter ; figure 13A : γl, 0.9% NaCl : indication de profondeur excessive ; figure 13'A : γ2, 0.9% NaCl : aucun résultat).

Or, sur ces mêmes modèles complexes β & γ de canaux radiculaires ramifiés β1-β2 et γ1-γ2, le procédé de mesure de profondeur de position donne précisément des mesures cohérentes M de la position en profondeur de l'extrémité de chacun des deux canaux radiculaires β1-β2 ou γ1-γ2, avec une faible erreur relative et n'indique pas de mesure excessive de profondeur majorée ou surestimée qui se situerait au-delà de la position réelle X de l'extrémité du canal de racine correspondant.

De façon particulièrement avantageuse, le procédé de mesure de position en profondeur de l'apex, c'est-à-dire de l'extrémité du canal radiculaire d'une dent permet de résoudre des canaux radiculaires de dents complexes présentant des bifurcations et/ou des aberrations. Le procédé permet de mesurer la profondeur M de chacun des canaux radiculaires, avec une bonne précision et évitant des indications de mesures majorées qui se situeraient au-delà de l'apex X dans le ligament sous la racine de la dent, ce qui satisfait l'objectif recherché par les dentistes pour leurs patients.

Le dernier volet de la campagne de mesures consiste à vérifier l'application du procédé de mesure de profondeur de l'apex sur des dents réelles.

Les figures 14A, 14B et 14C illustrent une série de courbes de mesure et de résultats obtenus sur une dent réelle Δ avec les choix des couples de fréquences déterminés, tels qu'indiqués précédemment, et en présence, respectivement, de solutions irrigantes de NaCl à 0,9%, et de NaClO à 2,5% et 5%.

Comme visible sur la figure 14A, le procédé de mesure de profondeur d'apex donne d'aussi bonnes mesures M de profondeur de la constriction apicale de la dent Δ, en présence de solution de chlorure de sodium (NaCl à 0,9%), avec une excellente précision de l'ordre d'un pourcent.

Et surtout, de façon particulièrement avantageuse, en présence de solution désinfectante conductrice à base d'hypochlorite de sodium (fig. 14B : 2,5% NaClO et fig. 14C : 5% NaClO), alors que le dispositif AD localisateur d'apex disponible antérieurement ne donne aucune indication de mesure (AD=0 "erreur-100%"), le procédé selon l'invention permet d'obtenir d'excellentes mesures de position en profondeur de l'apex de la dent réelle Δ avec une précision en deçà du pourcent (erreur inférieure à 1% et estimée à moins de 0,1% ou 0,6%).

De façon plus générale, il apparaît que le choix des valeurs de fréquences (f,F) est déterminant pour que le point de coïncidence M des deux niveaux d'amplitude absolue des signaux alternatifs mesurés aux deux fréquences inférieure et supérieure f et F corresponde exactement à la position réelle X de l'apex et pour obtenir des mesures précises M de position de profondeur de l'apex à l'extrémité du canal radiculaire d'une dent.

Les figures 15 à 18 représentent des diagrammes regroupant de manière synthétique l'erreur εₑᵣᵣ des mesures M de profondeur d'apex en fonction de la fréquence supérieure F utilisée en appliquant le procédé de localisation d'apex, selon l'invention.

Chacun des diagrammes 15 à 18 indique le taux d'erreur εₑᵣᵣ de chaque mesure M de profondeur d'apex selon l'invention, en fonction de la valeur de fréquence supérieure F sélectionnée produite par le générateur de fréquence GF, la valeur de fréquence inférieure f étant fixée ici à une valeur de fréquence basse f=100 Hz.

L'erreur de mesure εₑᵣᵣ est l'écart entre la mesure M de la position du point de coïncidence obtenue selon l'invention et la profondeur réelle X de l'extrémité du canal radiculaire mesurée métriquement sur des modèles dentaires α, β, γ ou sur des specimens de dents Δ.

Le taux d'erreur εₑᵣᵣ est exprimé en pourcentage par rapport à la longueur exacte de chaque canal radiculaire d'un modèle dentaire α, β, γ ou d'une dent réelle Δ, c'est-à-dire en pourcent de la profondeur réelle de l'apex.

La figure 15 regroupe les valeurs d'erreur de profondeur relevées sur les mesures de profondeur effectuées sur le modèle α de canal de racine de dent de la figure 7 dont plusieurs résultats de mesure ont été indiqués sur les figures 10A à 11B.

La figure 16 indique les valeurs d'erreurs des mesures de profondeur obtenues sur le modèle de canal dentaire β de la figure 8 (résultats de mesure illustrés sur les figures 12A-12'A et 12C-12'C).

La figure 17 indique les valeurs d'erreurs εₑᵣᵣ des mesures de profondeur M effectuées sur le modèle de canal dentaire γ de la figure 9 (cf. résultats de mesure indiqués sur les figures 13A à 13'C).

La figure 18 indique les valeurs d'erreur εₑᵣᵣ relevées sur une série de mesures de profondeur M effectuées sur six dents réelles (non représentés) et dont les figures 14A à 14C donnent des résultats de mesure.

Ces mesures de profondeur ont été effectuées suivant l'invention, avec une fréquence inférieure de valeur f = 100Hz et des valeurs de fréquence supérieure F prenant des valeurs déterminées choisies entre 200 kHz et 5MHz, notamment à des valeurs de fréquence de 500kHz, 1 MHz, 2MHz etc, donc englobant toute la bande de fréquence conventionnelle numéro six, qui couvre les fréquences allant de 300 kHz à 3 MégaHertz, en s'étendant le cas échéant dans les deux bandes voisines numéro cinq (30 kHz à 300 kHz, autour de 10⁵Hz) et numéro sept (3 MHz à 30 MHz, autour de 10⁷ Hz), conventionnellement.

Il apparaît que dans de telles bandes de fréquences, on peut obtenir de faibles erreurs de mesure εₑᵣᵣ, nettement inférieures à dix pourcents et même inférieure ou de l'ordre d'un ou de quelques pourcents. Plus précisément, l'erreur εₑᵣᵣ de mesure décroît régulièrement avec l'augmentation de la valeur de fréquence supérieure F et passe de valeurs d'erreur positive εₑᵣᵣ>0 pour des fréquences F comprises entre 200 kHz et 500 kHz, à des valeurs d'erreur négative εₑᵣᵣ<0 pour des fréquences F de l'ordre de 1 ou 2 MHz à 5MHz et plus.

Bien sûr, on cherche à minimiser l'erreur de mesure εₑᵣᵣ et en outre, comme annoncé précédemment, il est préférable pour les praticiens d'avoir une erreur de mesure négative εₑᵣᵣ<0, c'est-à-dire d'obtenir des mesures M de profondeur d'apex légèrement minorées (i.e. P sous estimées : M<X=100%), plutôt que de risquer d'avoir une erreur positive εₑᵣᵣ>0 et s'exposer à dépasser la position réelle du terminus apical.

D'après les diagrammes des figures 15 à 18, il apparaît ainsi que d'après les exemples de mise en oeuvre expérimentale de l'invention, tels qu' exposés dans la présente :
- En présence d'une solution aqueuse saline commune : (0,9% de NaCl), la position de l'apex X concorde avec le point M de coïncidence ou de croisement C du premier niveau d'amplitude du signal alternatif établi à la fréquence inférieure f (f=100 Hz) avec le second niveau d'amplitude du signal mesuré à une fréquence supérieure F comprise dans la bande de 200 kHz à 2 MégaHertz ou dans la bande conventionnelle numéro six (*i*.*e*. entre 300 kHz et 3 MHz) et, plus précisément, près des fréquences F de 500 kHz à 1 MHz ou dans les octaves de fréquences voisines *(i.e.* de 250 à 500 kHz, de 500 kHz à 1 MHz et/ou de 1 MHz à 2 MHz).
- En présence d'une solution aqueuse désinfectante à base d'hypochlorite de sodium (Soluté de Dakin ; NaClO à 2,5% ou 5%), la position X de l'apex correspond au point M de croisement ou de coïncidence C du premier niveau d'amplitude de signal établi à la fréquence inférieure f (f ∼ 100 Hz) avec un second niveau d'amplitude de signal obtenu à une fréquence supérieure F comprise dans la bande conventionnelle numéro six ou plus (de 300 kHz à 3MHz et plus), notamment dans une bande de fréquences F allant de 500 kHz à 5MHz et plus précisément dans l'octave des fréquences comprises entre 1 MégaHertz et 2 MégaHertz ou éventuellement dans l'une et/ou l'autre des octaves de fréquence voisines (i.e. de 0,5 MHz à 1 MHz et/ou de 2 MHz à 4 MHz).

La figure 18 montre que ces résultats et l'affinage de la plage des fréquences sélectionnées pour les mesures sont cohérents et en concordance avec toute une série de résultats de mesures effectuées sur des dents réelles. Il apparaît que le choix du couple de fréquences (f,F) permettant de détecter le point de coïncidence C des deux niveaux d'amplitudes If et IF et de mesurer la profondeur de l'apex, s'effectue en fonction de la nature de la solution irrigante :
- en présence de NaCl à 0,9%, la fréquence supérieure F est de préférence choisie dans la gamme de fréquence de 200 kHz à 5 MHz, notamment dans la bande conventionnelle numéro six (fréquence de 300 kHz à 3MHz) et plus précisément dans l'octave des fréquences d'environ 0,5 MHz à 1 MHz ou dans l'une et/ou l'autre des octaves de fréquences voisines (de 0,25 MHz à 0,5 MHz et/ou de 1 MHz à 2 MHz).
- En présence de solution antiseptique à base d'hypochlorite de sodium, la profondeur de l'apex correspond au point de coïncidence ou de croisement entre le premier niveau établi à la fréquence inférieure f d'environ 100 Hz et un second niveau mesuré à une fréquence supérieure F choisie dans une bande de fréquence entre 500 kHz et 5 MegaHertz ou dans la bande conventionnelle numéro six et plus précisément aux environs d'une octave de fréquence comprise entre 1 MHz et 2 MHz et/ou dans l'une et/ou l'autre des octaves de fréquences voisines (de 0,5 MHz à 1 MHz et/ou de 2 MHz à 4 MHz).

Selon l'invention, on peut réaliser ainsi un système de localisation d'apex, disposant d'une commande de calibre fréquentiel, permettant de régler la valeur de fréquence supérieure F sur une valeur calibrée parmi un ensemble de valeurs de fréquences présélectionnées, par exemple sur une valeur choisie parmi un ensemble de valeurs de fréquences, tel que {0,5 MHz ; 1 MHz ; 2 MHz ; 5 MHz} ou d'autres ensembles de fréquences approchantes, choisies dans les octaves ou les bandes de fréquences voisines. Une telle commande de calibre fréquentiel peut alternativement porter sur le choix de la valeur de fréquence inférieure f. On peut également prévoir deux commandes de calibres fréquentiels, l'une portant sur le choix de la fréquence inférieure f, l'autre sur le choix de la fréquence supérieure F. De telles dispositions permettent de faciliter les opérations du praticien qui peut actionner la commande calibrée en se référant uniquement à la nature de la solution irrigante qu'il injecte dans le canal radiculaire.

De préférence les commandes de sélection de fréquence pour exciter le circuit à la première fréquence inférieure et à la seconde fréquence supérieure, les mesures des niveaux d'amplitude absolue de l'intensité des signaux électriques fréquentiels dans ledit circuit et la détection du point de coïncidence sont effectuées automatiquement, au sein du dispositif, par un microprocesseur ou un microcontrôleur ou, plus généralement, par une unité de calcul programmée à cet effet, de façon à émettre un signal (sonore ou lumineux), pour prévenir le praticien, lorsque le premier niveau mesuré à la première fréquence inférieure n'est plus supérieur au second niveau de grandeur électrique du signal alternatif mesuré à la seconde fréquence supérieure.

Il apparaît en effet que, à la différence de l'enseignement des documents US-5,080,586 et US-5,096,419 (cf. fig. 3 et 4), dans le couple de fréquences opposées (f,F) mises en oeuvre selon l'invention, la fréquence inférieure f et la fréquence supérieure F sont sélectionnées de sorte que, dans la phase initiale I, au début de l'insertion de la pointe de la sonde au début du canal radiculaire CR ou de la partie coronaire de la dent, le premier niveau d'amplitude If=I1 établi à la fréquence inférieure f est supérieur, nettement supérieur, au second niveau d'amplitude IF=I2 établi à la seconde fréquence supérieure F.

Selon une réalisation alternative, on peut localiser l'apex en procédant comme suit :
- tant que le premier niveau If d'amplitude absolue de l'intensité du signal alternatif établi à la fréquence inférieure f est supérieur au second niveau IF [id.] établi à la fréquence supérieure F, l'extrémité de la sonde n'a pas atteint, ni dépassé, la position de l'apex X et le résultat du test de détection est négatif (pas de signal de détection d'apex)
- dès que le premier niveau d'amplitude absolue If établi à la fréquence inférieure f n'est pas, ou n'est plus, supérieur au second niveau IF établi à la fréquence supérieure F (alors le second niveau IF->I4 est supérieur ou sensiblement égal au premier niveau If->I3), le test de détection est positif et les moyens de détection du dispositif peuvent déclencher l'émission d'un signal pour prévenir le praticien.

Les valeurs des fréquences inférieure et supérieure (f, F) indiquées dans la présente sont données uniquement à titre d'exemples de réalisation non limitatifs, d'autres couples de fréquences pouvant être déterminés expérimentalement, par exemple en modifiant la valeur de la première fréquence inférieure f, notamment pour choisir d'autres valeurs de fréquence inférieure dans la bande radio n°2 ou 3 ou dans des bandes de fréquence inférieures à 100 Hz ou à la bande n°2, et/ou en sélectionnant d'autres valeurs de fréquences supérieures F, notamment d'autres fréquences supérieures F appariées avec de telles valeurs de fréquence inférieure f.

## Revendications

1. Dispositif de localisation d'apex pour déterminer une mesure (M) de position en profondeur de l'apex dans un canal de racine d'une dent (α,β,γ^{&}Δ),
comprenant une première électrode conductrice (E1) formant sonde endodontique (S) apte à être insérée dans le canal radiculaire (CR) d'une dent, une seconde électrode (E0) conformée pour être mise en contact conducteur électriquement avec une muqueuse buccale, des moyens (GF) générateurs de fréquences aptes à produire des signaux électriques alternatifs à plusieurs fréquences, et des moyens (AM) de mesure de grandeur de signaux électriques alternatifs dans un circuit comprenant ledit générateur de fréquences, la première électrode-sonde insérée dans le canal radiculaire et la seconde électrode au contact de la muqueuse buccale, le dispositif comportant en outre:
- des moyens de commande de sélection de fréquence pour exciter le circuit et mesurer les niveaux de grandeur (I) des signaux électriques alternatifs dans le circuit, respectivement, à une fréquence inférieure (f) et à une fréquence supérieure (F) ; **caractérisé par le fait que** le dispositif comporte
- des moyens pour détecter un point de coïncidence (C) où deux niveaux respectifs (If, IF) de la grandeur électrique (I) mesurée aux dites fréquences inférieure et supérieure (f, F) se croisent, lesdites fréquences (f, F) inférieure et supérieure étant suffisamment éloignées pour qu'un tel point de coïncidence (C) existe, et telles que ledit point de coïncidence (C, M) correspond à la position (X) de l'apex.

2. Dispositif de localisation d'apex selon la revendication 1, **caractérisé par le fait que** les moyens (AM) de mesure sont agencés pour mesurer des niveaux d'amplitude des signaux électriques appliqués au circuit et/ou de l'intensité (I) du courant traversant les électrodes (E0-E1).

3. Dispositif de localisation d'apex selon la revendication 2, **caractérisé par le fait que** les moyens (AM) de mesure sont agencés pour mesurer des valeurs absolues d'amplitude de tension (U=Rm.I) des signaux électriques aux bornes d'une résistance (Rm) en série avec les électrodes (E0-E1-S).

4. Dispositif de localisation d'apex selon l'une des revendications 1 à 3, **caractérisé par le fait que** les moyens de commande de sélection de fréquence sont agencés pour sélectionner la fréquence inférieure (f) et la fréquence supérieure (F) de sorte que, dans une phase initiale (I) correspondant au commencement de l'insertion de l'extrémité de l'électrode-sonde endodontique (E1-S) au début du canal radiculaire (CR), le premier niveau (If=I1) mesuré à la fréquence inférieure (f) est supérieur au second niveau (IF=I2) mesuré à la fréquence supérieure (F).

5. Dispositif de localisation d'apex selon l'une des revendications 1 à 4, **caractérisé par le fait que** les moyens de commande de sélection de fréquence sont agencés pour sélectionner les fréquences (f, F) inférieure et supérieure dans des bandes de fréquences opposées (BF, HF), distinctes et/ou distantes, i.e. non adjacentes.

6. Dispositif de localisation d'apex selon l'une des revendications 1 à 5, **caractérisé par le fait que** les moyens de commande de sélection de fréquence sont agencés pour sélectionner lesdites fréquences (f, F) inférieure et supérieure de sorte qu'elles sont séparées par un ou plusieurs ordres de magnitude, et/ou de préférence de sorte que ladite fréquence supérieure (F) est d'au moins deux, trois ou quatre ordres de magnitude supérieure à ladite fréquence inférieure (f).

7. Dispositif de localisation d'apex selon l'une des revendications 1 à 6, **caractérisé par le fait que** les moyens de commande de sélection de fréquence sont agencés pour sélectionner ladite fréquence inférieure (f) dans une bande de Basses Fréquences (BF) et ladite fréquence supérieure (F) dans une bande de Hautes Fréquences (HF).

8. Dispositif de localisation d'apex selon l'une des revendications 1 à 7, **caractérisé par le fait que** les moyens de commande de sélection de fréquence sont agencés pour sélectionner ladite fréquence inférieure (f) et ladite fréquence supérieure (F) de sorte qu'elles sont situées respectivement dans deux gammes de fréquences opposées (BF, HF) de part et d'autre d'une gamme de fréquences incluant au moins la bande conventionnelle numéro quatre (T.B.F. ou VLF ou B.O.hm ou n°4) couvrant les fréquences de trois kiloHertz à trente kiloHertz (3-30 kHz).

9. Dispositif de localisation d'apex selon l'une des revendications 1 à 8, **caractérisé par le fait que** les moyens de commande de sélection de fréquence sont agencés pour sélectionner ladite fréquence inférieure (f) de sorte que la fréquence inférieure (f) est inférieure à 950 Hertz, de préférence, inférieure à 500 Hertz.

10. Dispositif de localisation d'apex selon l'une des revendications 1 à 9, **caractérisé par le fait que** les moyens de commande de sélection de fréquence sont agencés pour sélectionner ladite fréquence supérieure (F) de sorte que la fréquence supérieure (F) est supérieure à 9500 Hertz, de préférence, supérieure à 95 kHz.

11. Dispositif de localisation d'apex selon l'une des revendications 1 à 10, **caractérisé par le fait que** les moyens de commande de sélection de fréquence sont agencés pour sélectionner la fréquence inférieure (f) dans une bande de fréquence conventionnelle numéro deux ou inférieur, *i*.*e*. comprise entre 300 Hertz et 30 Hertz ou moins.

12. Dispositif de localisation selon l'une des revendications 1 à 11, **caractérisé par le fait que** les moyens de commande de sélection de fréquence sont agencés pour sélectionner la fréquence supérieure (F) dans une bande de fréquence conventionnelle numéro six ou de numéro plus élevé, *i*.*e*. comprise entre 300 kHz et 3 MHz, voire plus.

13. Dispositif de localisation selon l'une des revendications 1 à 12, **caractérisé par le fait que** les moyens de commande de sélection de fréquence sont agencés pour sélectionner la fréquence inférieure (f) dans une bande de fréquence d'une dizaine de Hertz à quelques centaines de Hertz, de préférence autour d'une valeur de 100 Hertz, et pour sélectionner la fréquence supérieure (F) dans une bande de fréquence de l'ordre d'un demi ou d'un MégaHertz à cinq ou dix MégaHertz, les moyens de commande de sélection de fréquence étant de préférence agencés pour sélectionner la fréquence supérieure (F) sur une valeur choisie parmi un groupe de plusieurs valeurs calibrées autour de 0,5 MHz - 1 MHz-2MHz-5 MHz, en fonction des conditions électrolytiques régnant dans le canal radiculaire (CR), notamment de la présence de solutions aqueuses ioniques conductrices, telles que la présence de liquide physiologique ou de solution saline commune de chlorure de sodium (NaCl) ou la présence de liquide de Dakin ou de solution désinfectante à base d'hypochlorite de sodium (NaClO).

## Patentansprüche

1. Apexlokalisationsvorrichtung zur Bestimmung eines Maßes (M) der Position in der Tiefe des Apex in einem Zahnwurzelkanal (α, β, γ und Δ), welche eine erste Leitelektrode (E1) umfasst, welche eine endodontische Sonde (S) bildet, die zum Einsetzen in den Wurzelkanal (CR) eines Zahns geeignet ist, eine zweite Elektrode (E0), welche dazu ausgelegt ist, in elektrisch leitenden Kontakt mit einer Mundschleimhaut gebracht zu werden, Mittel (GF) zur Erzeugung von Frequenzen, welche geeignet sind, elektrische Wechselsignale mit mehreren Frequenzen zu erzeugen, und Mittel (AM) zum Messen der Stärke elektrischer Wechselsignale in einem Schaltkreis, welcher den genannten Frequenzgenerator, die in den Zahnwurzelkanal eingesetzte erste Elektrodensonde und die in Kontakt mit der Mundschleimhaut befindliche zweite Elektrode umfasst, wobei die Vorrichtung ferner Folgendes umfasst:
- Steuerungsmittel zur Wahl der Frequenz, um den Schaltkreis anzuregen und das Niveau der Stärke (I) der elektrischen Wechselsignale in dem Schaltkreis, jeweils bei einer niedrigen Frequenz (f) und einer hohen Frequenz (F), zu messen;
**dadurch gekennzeichnet, dass** die Vorrichtung Folgendes aufweist:
- Mittel zum Erfassen eines Überschneidungspunktes (C), an dem jeweils zwei Niveaus (If, IF) der elektrischen Stärke (I), gemessen bei der genannten niedrigen und hohen Frequenz (f, F), einander kreuzen, wobei die genannten niedrigen und hohen Frequenzen (f, F) ausreichend voneinander entfernt sind, so dass ein derartiger Überschneidungspunkt (C) existiert, und derart sind, dass der genannte Überschneidungspunkt (C, M) der Position (X) des Apex entspricht.

2. Apexlokalisationsvorrichtung nach Anspruch 1, **dadurch gekennzeichnet, dass** die Mittel (AM) zum Messen eingerichtet sind, um die Niveaus der Amplitude der an den Schaltkreis angelegten elektrischen Signale und/oder die Stärke (I) des durch die Elektroden (E0 - E1) laufenden Stroms zu messen.

3. Apexlokalisationsvorrichtung nach Anspruch 2, **dadurch gekennzeichnet, dass** die Mittel (AM) zum Messen eingerichtet sind, um absolute Werte der Spannungsamplitude (U = Rm.I) der elektrischen Signale an den Anschlüssen eines Widerstandes (Rm) in Serie mit den Elektroden (E0 - E1 - S) zu messen.

4. Apexlokalisationsvorrichtung nach einem der Ansprüche 1 bis 3, **dadurch gekennzeichnet, dass** die Steuerungsmittel zur Wahl der Frequenz eingerichtet sind, um die niedrige Frequenz (f) und die hohe Frequenz (F) so zu wählen, dass in einer anfänglichen Phase (I), welche dem Beginn des Einsetzens des Endes der endodontischen Elektrodensonde (E1 - S) am Anfang des Zahnwurzelkanals (CR) entspricht, das erste bei der niedrigen Frequenz (f) gemessene Niveau (If = I1) höher ist als das zweite bei der hohen Frequenz (F) gemessene Niveau (IF = I2).

5. Apexlokalisationsvorrichtung nach einem der Ansprüche 1 bis 4, **dadurch gekennzeichnet, dass** die Steuerungsmittel zur Wahl der Frequenz eingerichtet sind, um die niedrigen und hohen Frequenzen (f, F) aus einander gegenüberliegenden, separaten und/oder voneinander entfernten, d.h. nicht aneinander angrenzenden, Frequenzbändern (BF, HF) auszuwählen.

6. Apexlokalisationsvorrichtung nach einem der Ansprüche 1 bis 5, **dadurch gekennzeichnet, dass** die Steuerungsmittel zur Wahl der Frequenz eingerichtet sind, um die genannten niedrigen und hohen Frequenzen (f, F) so zu wählen, dass diese um eine oder mehrere Größenordnungen voneinander getrennt sind, und/oder vorzugsweise so, dass die genannte hohe Frequenz (F) um mindestens zwei, drei oder vier Größenordnungen höher ist als die genannte niedrige Frequenz (f).

7. Apexlokalisationsvorrichtung nach einem der Ansprüche 1 bis 6, **dadurch gekennzeichnet, dass** die Steuerungsmittel zur Wahl der Frequenz eingerichtet sind, um die genannte niedrige Frequenz (f) aus einem Tieffrequenzband (BF) und die genannte hohe Frequenz (F) aus einem Hochfrequenzband (HF) auszuwählen.

8. Apexlokalisationsvorrichtung nach einem der Ansprüche 1 bis 7, **dadurch gekennzeichnet, dass** die Steuerungsmittel zur Wahl der Frequenz eingerichtet sind, um die genannte niedrige Frequenz (f) und die genannte hohe Frequenz (F) so zu wählen, dass sie sich jeweils in zwei Frequenzbereichen (BF, HF) befinden, welche einander auf beiden Seiten eines Frequenzbereichs gegenüberliegen, der mindestens das konventionelle Band 4 (SLW oder VLF oder Längstwellen) einschließt, welches die Frequenzen von drei Kilohertz bis dreißig Kilohertz (3 - 30 kHz) abdeckt.

9. Apexlokalisationsvorrichtung nach einem der Ansprüche 1 bis 8, **dadurch gekennzeichnet, dass** die Steuerungsmittel zur Wahl der Frequenz eingerichtet sind, um die genannte niedrige Frequenz (f) so zu wählen, dass die niedrige Frequenz (f) niedriger als 950 Hertz, vorzugsweise niedriger als 500 Hertz, ist.

10. Apexlokalisationsvorrichtung nach einem der Ansprüche 1 bis 9, **dadurch gekennzeichnet, dass** die Steuerungsmittel zur Wahl der Frequenz eingerichtet sind, um die genannte hohe Frequenz (F) so zu wählen, dass die hohe Frequenz (F) höher als 9500 Hertz, vorzugsweise höher als 95 kHertz, ist.

11. Apexlokalisationsvorrichtung nach einem der Ansprüche 1 bis 10, **dadurch gekennzeichnet, dass** die Steuerungsmittel zur Wahl der Frequenz eingerichtet sind, um die niedrige Frequenz (f) aus einem konventionellen Frequenzband Nr. 2 oder niedriger, d.h. in einem Bereich zwischen 300 Hertz und 30 Hertz oder weniger, zu wählen.

12. Apexlokalisationsvorrichtung nach einem der Ansprüche 1 bis 11, **dadurch gekennzeichnet, dass** die Steuerungsmittel zur Wahl der Frequenz eingerichtet sind, um die hohe Frequenz (F) aus einem konventionellen Frequenzband Nr. 6 oder mit einer höheren Nummer, d.h. in einem Bereich zwischen 300 kHz und 3 MHz oder sogar mehr, zu wählen.

13. Apexlokalisationsvorrichtung nach einem der Ansprüche 1 bis 12, **dadurch gekennzeichnet, dass** die Steuerungsmittel zur Wahl der Frequenz eingerichtet sind, um die niedrige Frequenz (f) aus einem Frequenzband von etwa zehn Hertz bis zu einigen Hundert Hertz, vorzugsweise einen Wert von etwa 100 Hertz, zu wählen und um die hohe Frequenz (F) aus einem Frequenzband von ungefähr einem halben bis einem Megahertz bis fünf oder zehn Megahertz zu wählen, wobei die Steuerungsmittel zur Wahl der Frequenz vorzugsweise eingerichtet sind, um die hohe Frequenz (F) bei einem aus einer Gruppe aus mehreren kalibrierten Werten gewählten Wert um 0,5 MHz - 1 MHz - 5 MHz zu wählen
in Abhängigkeit von den im Zahnwurzelkanal (CR) herrschenden elektrolytischen Bedingungen, insbesondere von der Anwesenheit leitender wässriger ionischer Lösungen wie beispielsweise der Anwesenheit physiologischer Flüssigkeiten oder gewöhnlicher Natriumchlorid-Salzlösung (NaCl) oder der Anwesenheit von Dakin-Flüssigkeit oder von desinfizierender Lösung auf der Basis von Natriumhypochlorit (NaClO).

## Claims

1. Apex-locating device for determining a measurement (M) of the depth position of the apex in a root canal of a tooth (α, β, γ, Δ), comprising a first conductive electrode (E1) forming an endodontic probe (S) able to be inserted into the root canal (CR) of a tooth, a second electrode (E0) shaped to be brought into electrically conductive contact with an oral mucous membrane, frequency-generating means (GF) able to produce alternating electrical signals at a number of frequencies, and means (AM) for measuring the magnitude of alternating electrical signals in a circuit comprising the said frequency generator, the first probe electrode inserted into the root canal and the second electrode in contact with the oral mucous membrane, the device further comprising
frequency selection control means to excite the circuit and measure magnitude levels of the alternating electrical signals (I) in the circuit, respectively at a first lower frequency (f) and at a second higher frequency (F);
**characterised in that** the device comprises
means for detecting a point of coincidence (C) where two respective levels (If, IF) of the electrical magnitude (I) measured at the said lower and higher frequencies (f, F) cross, the said lower and higher frequencies (f, F) being sufficiently far apart for such a point of coincidence (C) to exist, and being such that the said point of coincidence (C, M) corresponds to the position (X) of the apex.

2. Apex-locating device as claimed in claim 1, **characterised in that** the said measuring means (AM) are arranged to measure amplitude levels of the electrical signals applied to the circuit and/or the intensity (I) of the current passing through the electrodes (E0-E1).

3. Apex-locating device as claimed in claim 2, **characterised in that** the said measuring means (AM) are arranged to measure absolute voltage amplitude values (U = Rm.I) of the electrical signals at the terminals of a resistor (Rm) in series with the electrodes (E0-E1-S).

4. Apex-locating device as claimed in any one of claims 1 to 3, **characterised in that** the frequency selection control means are arranged to select the lower frequency (f) and the higher frequency (F) so that in an initial phase (I), corresponding to the commencement of the insertion of the end of the endodontic probe electrode (E1-S) at the beginning of the root canal (CR), the first level (If=I1) measured at the lower frequency (f) is higher than the second level (IF = 12) measured at the higher frequency (F).

5. Apex-locating device as claimed in any one of claims 1 to 4, **characterised in that** the frequency selection control means are arranged to select the lower and higher frequencies (f, F) in opposing frequency bands (BF, HF) which are distinct and/or far apart, i.e. non-adjacent.

6. Apex-locating device as claimed in any one of claims 1 to 5, **characterised in that** the frequency selection control means are arranged to select the said lower and higher frequencies (f, F) so that they are separated by one or more orders of magnitude, and/or preferably the said higher frequency (F) is at least two, three or four orders of magnitude higher than the said lower frequency (f).

7. Apex-locating device as claimed in any one of claims 1 to 6, **characterised in that** the frequency selection control means are arranged to select the said lower frequency (f) in a low frequency band (BF) and the said higher frequency (F) in a high frequency band (HF).

8. Apex-locating device as claimed in any one of claims 1 to 7, **characterised in that** the frequency selection control means are arranged to select the said lower frequency (f) and the said higher frequency (F) so that they are located respectively in two opposing frequency ranges (BF, HF) on either side of a frequency range including at least the conventional number four band (VLF or hm.W.B. or no. 4) which covers the frequencies of three kilohertz to thirty kilohertz (3-30 kHz).

9. Apex-locating device as claimed in any one of claims 1 to 8, **characterised in that** the frequency selection control means are arranged to select the said lower frequency (f) so that the lower frequency (f) is lower than 950 hertz, preferably lower than 500 hertz.

10. Apex-locating device as claimed in any one of claims 1 to 9, **characterised in that** the frequency selection control means are arranged to select the said higher frequency (F) so that the higher frequency (F) is higher than 9500 hertz, preferably higher than 95 kHz.

11. Apex-locating device as claimed in any one of claims 1 to 10, **characterised in that** the frequency selection control means are arranged to select the lower frequency (f) in a conventional number two or lower frequency band, i.e. between 300 hertz and 30 hertz or less.

12. Apex-locating device as claimed in any one of claims 1 to 11, **characterised in that** the frequency selection control means are arranged to select the higher frequency (F) in a conventional number six or higher-number frequency band, i.e. between 300 kHz and 3 MHz, or more.

13. Apex-locating device as claimed in any one of claims 1 to 12, **characterised in that** the frequency selection control means are arranged to select the lower frequency (f) in a frequency band of about ten hertz to several hundred hertz, preferably around a value of 100 hertz, and to select the higher frequency (F) in a frequency band of the order of one half or one megahertz to five or ten megahertz, the frequency selection control means being preferably arranged to select the higher frequency (F) to a value selected among a group of several calibrated values around 0.5 MHz - 1 MHz - 2MHz - 5MHz depending on the electrolytic conditions prevailing in the root canal (CR), in particular of the presence of conductive aqueous ionic solutions such as the presence of physiological liquid or a common saline solution of sodium chloride (NaCl) or the presence of Dakin's liquid or a disinfectant solution based on sodium hypochlorite (NaClO).
